(19) Europäisches Patentamt
European Patent Office
Office européen des brevets

(11) **EP 1 633 467 B1**

(12) **EUROPÄISCHE PATENTSCHRIFT**

(45) Veröffentlichungstag und Bekanntmachung des Hinweises auf die Patenterteilung:
**22.02.2017 Patentblatt 2017/08**

(21) Anmeldenummer: **04739464.8**

(22) Anmeldetag: **29.05.2004**

(51) Int Cl.:
*B01J 8/10* *(2006.01)*      *B01J 19/28* *(2006.01)*

(86) Internationale Anmeldenummer:
**PCT/EP2004/005850**

(87) Internationale Veröffentlichungsnummer:
**WO 2004/108267 (16.12.2004 Gazette 2004/51)**

(54) **VERFAHREN ZUR THERMISCHEN BEHANDLUNG EINER KATALYTISCHEN AKTIVMASSE**

METHOD FOR THE THERMAL TREATMENT OF AN ACTIVE CATALYTIC MASS

PROCEDE DE TRAITEMENT THERMIQUE D'UNE MASSE ACTIVE CATALYTIQUE

(84) Benannte Vertragsstaaten:
**AT BE BG CH CY CZ DE DK EE ES FI FR GB GR HU IE IT LI LU MC NL PL PT RO SE SI SK TR**

(30) Priorität: **04.06.2003 DE 10325487**
**04.06.2003 US 475501 P**
**19.12.2003 DE 10360057**
**19.12.2003 US 530620 P**

(43) Veröffentlichungstag der Anmeldung:
**15.03.2006 Patentblatt 2006/11**

(73) Patentinhaber: **BASF SE**
**67056 Ludwigshafen am Rhein (DE)**

(72) Erfinder:
• **DIETERLE, Martin**
**68167 Mannheim (DE)**
• **PÖPEL, Wolfgang, Jürgen**
**64297 Darmstadt (DE)**
• **BERNDT, Silke**
**68165 Mannheim (DE)**
• **FELDER, Raimund**
**67434 Neustadt (DE)**
• **UNVERRICHT, Signe**
**68169 Mannheim (DE)**
• **MÜLLER-ENGEL, Klaus, Joachim**
**76297 Stutensee (DE)**

(56) Entgegenhaltungen:
**DE-A- 19 815 281**    **DE-C- 618 872**
**US-A- 5 719 101**    **US-A- 5 739 391**

EP 1 633 467 B1

**Beschreibung**

Vorliegende Erfindung betrifft ein Verfahren zur thermischen Behandlung der Vorläufermasse einer katalytischen Aktivmasse in einem von einem Gasstrom durchströmten Drehrohrofen.

Unter dem Begriff "katalytische Aktivmasse" sollen in dieser Schrift Feststoffe verstanden werden, die in chemischen Reaktionen, z.B. in chemischen Gasphasenreaktionen, zusätzlich zu den Reaktanden eingesetzt werden, um entweder die zur Durchführung der chemischen Reaktion erforderliche Temperatur zu mindern und/oder die Selektivität der Zielproduktbildung zu erhöhen. Dabei verläuft die chemische Reaktion in der Regel an der Oberfläche (Grenzfläche) der katalytischen Aktivmasse.

Beispiele für solche heterogen katalysierten Reaktionen, die prinzipiell sowohl in der Flüssigphase als auch in der Gasphase durchgeführt werden können, sind heterogen katalysierte Hydrierungen, Dehydrierungen und Oxidehydrierungen, aber auch partielle Oxidationen und partielle Ammoxidationen. Dabei kann die Aktivmasse sowohl in Pulverform als auch zu geometrischen Formkörpern geformt eingesetzt werden (letzteres kann z.B. durch Einbringen (Absorbieren) in die innere Oberfläche eines vorgeformten Trägerkörpers (man spricht dann von Trägerkatalysator), durch Aufbringen auf die äußere Oberfläche eines vorgeformten Trägerkörpers (man spricht dann von Schalenkatalysator) oder durch Verpressen (man spricht dann von Vollkatalysatoren erfolgen). Die Formgebung kann dabei bereits der Vorläufermasse oder erst der katalytischen Aktivmasse aufgeprägt werden.

Unter einer vollständigen Oxidation einer organischen Verbindung (z.B. eines gesättigten oder ungesättigten Kohlenwasserstoffs, eines Alkohols oder eines Aldehyds) wird in dieser Schrift verstanden, dass der in der organischen Verbindung insgesamt enthaltene Kohlenstoff in Oxide des Kohlenstoffs ($CO$, $CO_2$) umgewandelt wird.

Alle anderen Umsetzungen organischer Verbindungen mit Sauerstoff (einschließlich der Oxydehydrierungen) werden in dieser Schrift als Partialoxidationen betrachtet. Die partielle Ammoxidation unterscheidet sich von der partiellen Oxidation durch ein zusätzliches Beisein von Ammoniak.

Es ist nun allgemein bekannt, dass katalytische Aktivmassen, bereits geformt oder ungeformt, in der Regel dadurch erhältlich sind, dass man eine in der Regel katalytisch nicht oder allenfalls gemindert aktive Vorläufermasse erzeugt, die bereits geformt oder noch ungeformt sein kann, und diese bei erhöhter Temperatur einer spezifischen Gasatmosphäre aussetzt.

Beispielsweise beschreibt die DE-A 10211275 in ihren Ausführungsbeispielen die Aktivierung einer Vorläufermasse eines Dehydrierkatalysators bei erhöhter Temperatur (500°C) in wechselnden Gasströmen (Wasserstoff, Luft, Stickstoff) und ihre Verwendung in Katalysatoren für die heterogen katalysierte Dehydrierung von Kohlenwasserstoffen in der Gasphase.

In ähnlicher Weise beschreiben die Schriften EP-A 529853, EP-A 318295, EP-A 608838, WO 01/96270, EP-A 731077, EP-A 1260495, EP-A 1254709, EP-A 1192987, EP-A 962253, EP-A 1262235, EP-A 1090684, DE-A 19835247, EP-A 895809, DE-A 10261186, EP-A 774297, WO 02/24620, EP-A 668104, DE-A 2161450, EP-A 724481, EP-A 714700, DE-A 10046928 und DE-A 19815281 in unterschiedlichsten Gasatmosphären die thermische Behandlung von Vorläufermassen von Multielementoxidaktivmassen. Die dabei resultierenden Multielementoxidaktivmassen werden in diesen Schriften als katalytische Aktivmassen in Katalysatoren für die unterschiedlichsten heterogen katalysierten partiellen Gasphasenoxidationen und - ammoxidationen organischer Verbindungen empfohlen und eingesetzt.

Prinzipiell können solche thermischen Behandlungen in den unterschiedlichsten Ofentypen wie z.B. Hordenöfen, Drehrohröfen, Bandcalzinierer, Wirbelbettöfen oder Schachtöfen durchgeführt werden.

Von zunehmender Bedeutung ist dabei (vgl. WO 02/24620), dass die insgesamt thermisch zu behandelnde Vorläufermasse unter möglichst einheitlichen Bedingungen behandelt wird, um eine möglichst einheitlich beschaffene Gesamtmenge an Aktivmasse zu erhalten.

Beispielsweise eignen sich möglichst einheitlich beschaffene Mengen an Aktivmasse besser für heterogene Gasphasenkatalysen mit hoher Reaktandenbelastung der Aktivmasse, da sie besonders einheitliche thermische Reaktionsverhältnisse über einen Reaktorquerschnitt ermöglichen.

Vor diesem Hintergrund empfiehlt die WO 02/24620, die thermische Behandlung solcher Vorläufermassen mittels einer speziellen Bandcalciniervorrichtung durchzuführen. Nachteilig an einer Bandcalcination ist jedoch, dass sie an ruhenden Schüttungen der Vorläufermasse erfolgt. Innerhalb einer solchen ruhenden Schüttung bilden sich jedoch normalerweise Temperaturgradienten aus, die einer gleichmäßigen thermischen Behandlung über die insgesamt thermisch zu behandelnde Vorläufermasse entgegenwirken.

Demgegenüber bevorzugt wäre eine thermische Behandlung der Vorläufermasse in bewegter Schüttung, wie sie bei thermischen Behandlungen in Drehrohröfen vorliegt. Durch die fortwährende Bewegtheit der Vorläufermasse in Drehrohröfen bildet selbige eine sich stetig selbst homogenisierende Schüttung, innerhalb der beispielsweise eine Ausbildung von sogenannten "Neiß-Punkten" oder "Kalt-Punkten" ausbleibt (bei den meisten thermischen Behandlungen von Vorläufermassen laufen exotherme oder endotherme Prozesse ab, die zur Ausbildung von sogenannten "Heiß-Punkten" (Orte maximaler Temperatur innerhalb der thermisch behandelten Vorläufermasse) bzw. zur Ausbildung von sogenannten "Kalt-Punkten" (Orte minimaler Temperatur innerhalb der thermisch behandelten Vorläufermasse führen).

Sowohl zu hohe als auch zu niedere Temperaturen beeinträchtigen jedoch die katalytischen Eigenschaften der Aktivmasse.

Ein anderer Gesichtspunkt ist, dass mit der überwiegenden Anzahl von thermischen Behandlungen von Vorläufermassen thermische Zersetzungsprozesse von in der Vorläufermasse enthaltenen chemischen Bestandteilen einhergehen, wobei sich gasförmige Zersetzungsprodukte bilden, die sich auf die resultierende Aktivmassenqualität vorteilhaft oder nachteilig auswirken können. In beiden Fällen wäre eine Selbsthomogenisierung in bewegter Schüttung vorteilhaft.

Eine thermische Behandlung von Aktivmassenvorläufermasse im Drehrohrofen wird u.a. in den Schriften DE-A 19815281 (z.B. Beispiel 1), DE-A 10046928 (z.B. Herstellungsbeispiel 1) und EP-A 714700 (Ausführungsbeispiele) angeregt.

Üblicherweise wird eine solche thermische Behandlung im Drehrohrofen großtechnisch so durchgeführt, dass der Neigungswinkel des Drehrohres zur Horizontalen auf einen von Null verschiedenen Wert eingestellt wird. Die höchstliegende Stelle des Drehrohres bildet die Eintragsstelle der Vorläufermasse und die tiefstliegende Stelle bildet den Ort des Materialgutaustrags. Dabei wird das Drehrohr kontinuierlich betrieben. D.h., die thermisch zu behandelnde Vorläufermasse wird dem Drehrohr auf der einen Seite kontinuierlich zugeführt, im Drehrohr kontinuierlich von der höchstgelegenen zur tiefstgelegenen Stelle transportiert und dort kontinuierlich ausgetragen. Auf dem Weg durch das Drehrohr erfährt die Vorläufermasse normalerweise die thermische Behandlung.

Nachteilig ist, dass eine solche kontinuierliche Fahrweise nur vergleichsweise kurze Verweilzeiten des thermisch zu behandelnden Materialguts im Drehrohr zulässt.

Zur Einstellung der gewünschten Gasatmosphäre wird normalerweise im Gegenstrom zur geförderten Vorläufermasse ein entsprechender Gasstrom durch das Drehrohr geführt. Im einfachsten Fall kann dieser aus Luft, in anderen Fällen aber u.a. auch aus Wertgasen (z.B. reduzierend wirkenden Gasen wie Wasserstoff oder Ammoniak oder inerten Gasen wie z.B. Stickstoff) bestehen.

Nachteilig an der beschriebenen Verfahrensweise ist der vergleichsweise hohe Bedarf an solchen Gasen, die nach Verlassen des Drehrohres nicht weiter verwendet werden. Nachteilig ist auch, dass im Rahmen von im Materialgut durch thermische Zersetzung gebildete, günstig einwirkende, Gase mit dem Gasstrom ausgetragen werden und ihre vorteilhafte Wirkung nicht weiter entfalten können (z.B. aus $NH_4^+$ gebildeter $NH_3$, oder aus $NO_3$ gebildetes $NO_2$, oder aus $CO_3^{2-}$ gebildetes $CO_2$ bzw. $CO$). Eine solche vorteilhafte Wirkung kann z.B. in einer reduzierenden Wirkung bestehen.

Die im im Drehrohr befindlichen Materialgut erwünschte Temperatur wird normalerweise auf indirektem Weg erzeugt, indem die Drehrohrwand von außen auf bestimmte Temperatur gebracht wird.

Um dabei im Drehrohr ausgeprägte radiale und axiale Temperaturgradienten zu vermeiden, wäre es wünschenswert, den durch das Drehrohr geführten Gasstrom auf die für das Materialgut im Drehrohr erwünschte Temperatur vorerwärmt ins Drehrohr zu führen.

Normalerweise würde dieser Wärmeinhalt des Gasstroms beim Verlassen des Drehrohres in nachteiliger Weise nicht weitergenutzt.

Die Aufgabe der vorliegenden Erfindung bestand angesichts dieses Standes der Technik darin, ein Verfahren zur thermischen Behandlung der Vorläufermasse einer katalytischen Aktivmasse in einem von einem Gasstrom durchströmten Drehrohrofen zur Verfügung zu stellen, das den Nachteilen der Verfahren des Standes der Technik weitgehend abhilft.

Demgemäss wurde ein Verfahren zur thermischen Behandlung der Vorläufermasse einer katalytischen Aktivmasse gemäss Anspruch 1 gefunden.

Der durch den Drehrohrofen strömende Gasstrom kann beim erfindungsgemäßen Verfahren > 0 bis 500 Nm³/h (bzw. bis 300 Nm³/h im Fall von pulverförmigen Vorläufermassen) betragen. Häufig wird der durch den Drehrohrofen geführte Gasstrom ≥ 50 bis 500 (bzw. 300) Nm³/h und meist 100 bis 300 Nm³/h betragen.

Zur Ausübung des erfindungsgemäßen Verfahrens bedarf es einer geeigneten Drehrohrofenvorrichtung. Im Folgenden soll die Ausgestaltung einer solchen aufgezeigt werden, ohne damit das erfindungsgemäße Verfahren oder die dazu verwendbaren Drehrohrofenvorrichtungen in irgendeiner Form zu beschränken.

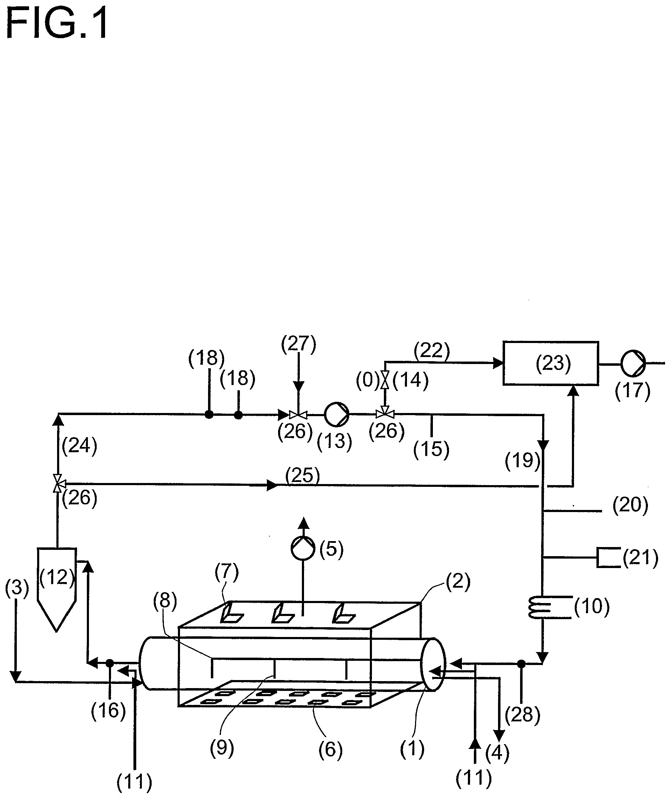

Eine schematische Darstellung dieser für das erfindungsgemäße Verfahren beispielhaft geeigneten (und im Ausführungsbeispiel verwendeten) Drehrohrofenvorrichtung zeigt die Figur 1, auf die sich auch die nachfolgend verwendeten Bezugsziffern beziehen. Die Regelungen erfolgen vorzugsweise Computer gesteuert.

Zentrales Element einer solchen zur Durchführung des erfindungsgemäßen Verfahrens geeigneten Drehrohrofenvorrichtung ist das Drehrohr (1).

Es kann z.B. 4000 mm lang sein und einen Innendurchmesser von 700 mm aufweisen. Dabei kann es aus Edelstahl 1.4893 gefertigt sein und eine Wanddicke von 10 mm aufweisen (selbstredend kommen für das erfindungsgemäße Verfahren auch Drehrohrlängen von bis zu 15 m, häufig von bis zu 12 m in Betracht; in der Regel wird die Drehrohrlänge beim erfindungsgemäßen Verfahren ≥ 1 m, in der Regel ≥ 4 m betragen; der Innendurchmesser liegt dabei üblicherweise bei 20 cm bis 2 m, häufig bei 20 cm bis 1 m).

In zweckmäßiger Weise können auf der Innenwand des Drehrohrofens sogenannte Hublanzen angebracht sein. Sie dienen primär dem Zweck, die im Drehrohrofen thermisch zu behandelnde Vorläufermasse (das thermisch zu behandelnde Materialgut) anzuheben und so die Selbsthomogenisierung in bewegter Schüttung zu fördern. Bezogen auf die übrigen beispielhaft genannten Dimensionen der beispielhaft beschriebenen erfindungsgemäß geeigneten Drehrohrvorrichtung (die sich alle aufeinander beziehen und im Ausführungsbeispiel angewendet wurden) kann die Höhe der Hublanzen 5 cm betragen. Prinzipiell kann sich eine einzelne Hublanze auf die gesamte Länge des Drehrohres erstrecken. In zweckmäßiger Weise wird sich eine Hublanze jedoch nur auf eine Teillänge der Drehrohrlänge erstrecken. In der beispielhaften Ausführungsvariante können sie z.B. eine Länge von 23,5 cm aufweisen. In diesen Fällen ist es erfindungsgemäß vorteilhaft, wenn auf ein und derselben Höhe des Drehrohrofens um den Umfang äquidistant (z.B. jeweils 90°) mehrere (ein Multiple) Hublanzen (z.B. vier = ein Quadrupel) angebracht sind. Längs des Drehrohres befinden sich anwendungstechnisch vorteilhaft mehrere solcher Multiple. In der beispielhaften Ausführungsvariante befinden sich längs des Drehrohres acht solcher Quadrupel (jeweils im Abstand von 23,5 cm). Die Hublanzen zweier benachbarter Multiple (Quadrupel) sitzen am Umfang des Drehrohres vorzugsweise gegeneinander versetzt. Am Anfang und am Ende des Drehrohrofens (erste und letzte 23,5 cm in der beispielhaften Ausgestaltung) befinden sich vorteilhaft keine Hublanzen.

Die Umdrehungsgeschwindigkeit des Drehrohres kann vorteilhaft variabel eingestellt werden. Typische Umdrehungszahlen liegen bei > 0 bis 5 oder 3 Umdrehungen pro Minute. Das Drehrohr ist in zweckmäßiger Weise sowohl links- als auch rechtsdrehbar. Zum Beispiel bei Rechtsdrehung verweilt das Materialgut im Drehrohr, z.B. bei Linksdrehung wird die thermisch zu behandelnde Vorläufermasse durch die Drehrohrneigung vom Eintrag (3) zum Austrag (4) gefördert und mittels einer Austragshilfe (z.B. einer Schaufel) aus dem Drehrohr entnommen.

Der Neigungswinkel des Drehrohres zur Horizontalen kann vorteilhaft variabel eingestellt werden. Typische Bereichswerte liegen bei 0° bis 4° bzw. 2°. Im diskontinuierlichen Betrieb liegt er faktisch bei 0°. Im kontinuierlichen Betrieb befindet sich die tiefstliegendste Stelle des Drehrohres beim Materialgutaustrag.

Der Materialguteintrag wird zweckmäßig über eine Zellenradschleuse volumenkontrolliert oder über eine Waage massenkontrolliert durchgeführt. Der Materialgutaustrag wird, wie bereits erwähnt, über die Drehrichtung des Drehrohrs gesteuert.

Bei diskontinuierlichem Betrieb des wie vorstehend dimensionierten Drehrohres (das zur Ausübung des erfindungsgemäßen Verfahrens, wie bereits erwähnt, auch größer oder kleiner dimensioniert sein könnte) kann eine Materialgutmenge von 200 bis 600 kg thermisch behandelt werden. Sie befindet sich dabei normalerweise ausschließlich im beheizten Teil des Drehrohres.

Um, wie erfindungsgemäß erforderlich, wenigstens eine Teilmenge des den Drehrohrofen durchströmenden Gasstroms im Kreis führen zu können, benötigt die erfindungsgemäß zu verwendende Drehrohrofenvorrichtung eine dazu erforderliche Kreisleitung (im einfachsten Fall ein Rohrleitsystem). Während dieses statisch eingerichtet ist, ist das Drehrohr in notwendiger Weise beweglich (Rotation) angebracht.

Um am Drehrohreintritt und am Drehrohraustritt jeweils das ruhende und das rotierende Element miteinander zu verbinden, können entweder Kugellager oder Spalte angewendet werden, die durch Graphit- oder Teflonringe abgedichtet sind (Graphit- bzw. Teflonpressdichtungen). Wegen ihrer Hochtemperaturbeständigkeit sind Graphitpressdichtungen bevorzugt.

Zweckmäßigerweise verjüngt sich dabei das Drehrohr an seinem Anfang und an seinem Ende und ragt in das zu- bzw. wegführende Rohr der Kreisleitung hinein.

Diese Verbindungen werden vorteilhaft mit Sperrgas gespült. Die beiden Spülströme (11) ergänzen den durch das Drehrohr geführten Gasstrom am Eintritt des Gasstroms in das Drehrohr und am Austritt des Gasstroms aus dem Drehrohr. Die chemische Zusammensetzung der Spülströme kann an die im Drehrohr für die thermische Behandlung gewünschte Temperatur angepasst werden. Beispielsweise kann als Sperrgas Luft verwendet werden. Insbesondere bei bei höheren Temperaturen ausgeführten thermischen Behandlungen wird jedoch bevorzugt Inertgas (z.B. Stickstoff) als Sperrgas verwendet, erzeugt dieses doch neben der Sperrwirkung gleichzeitig einen Oxidationsschutz. Bevorzugt wird die Sperrgasmenge niedrig gehalten. Ein einzelner solcher Spülstrom wird daher zweckmäßig > 0 bis 50 $Nm^3$/h, bevorzugt 1 bis 50 $Nm^3$/h betragen.

Die Sperrwirkung ist unter anderem wichtig, um zu vermeiden, dass von der im Drehrohr selbst herrschenden Gasatmosphäre, die häufig auch toxisch nicht völlig unbedenkliche Bestandteile (z.B. $CO_2$, $NO_x$, $NH_3$, CO, $SO_2$, Acetonitril) enthält, an der Verbindungsstelle statisch/drehend nichts in die Umgebungsatmosphäre gelangt.

Die Förderung des Kreisgasstroms (die Gaszirkulation) erfolgt mittels eines Kreisgasverdichters (13) (Ventilators, z.B. eines solchen vom Typ KXE 160-004030-00, der Fa. Konrad Reitz GmbH), der in Richtung des aus dem Drehrohr ausströmenden Gasstroms ansaugt und in die andere Richtung drückt. Unmittelbar hinter dem Kreisgasverdichter liegt der Gasdruck in der Regel oberhalb Atmosphärendruck (d.h., oberhalb von einer Atmosphäre). Hinter dem Kreisgasverdichter befindet sich ein Kreisgasauslaß (über ein Regelventil (14) kann Kreisgas ausgeschleust werden, das über den Abgasverdichter(17) (Ventilator; z.B. vom Typ T100/315-R3/500, von der Fa. Meidinger) angesaugt wird). Hinter der Auslassstelle für das Kreisgas befindet sich erfindungsgemäß zweckmäßig ein Druckminderer (15), z.B. eine Blende (Querschnittsverjüngung; z.B. um etwa einen Faktor 3). Dieser erschließt in einfacher Weise die Möglichkeit,

4

auf der Ansaugseite des Kreisgasverdichters mehr Gas anzusaugen als auf der Druckseite des Kreisgasverdichters weiterzugeben.

Auf diese Art und Weise kann im Drehrohr ein leichter Unterdruck eingestellt werden. D.h., der Druck des das Drehrohr durchströmenden Gasstroms kann beim Verlassen des Drehrohrs unterhalb des Umgebungsdrucks des Drehrohrs liegen. Diese Maßnahme trägt zusätzlich dazu bei, dass von der Gasatmosphäre im Drehrohr nichts nach außen dringt. Im Drehrohr kann aber auch relativ zum Umgebungsdruck ein leichter Überdruck eingestellt werden. Häufig wird man den Druck unmittelbar hinter dem Ausgang des Gasstroms aus dem Drehrohr auf einen Wert im Bereich von +1,0 mbar oberhalb bis zu -1,2 mbar unterhalb des Außendrucks einstellen. D.h., der Druck des das Drehrohr durchströmenden Gasstroms kann beim Verlassen des Drehrohrs unterhalb des Umgebungsdrucks des Drehrohrs liegen. Dies ist erfindungsgemäß bevorzugt.

Die Einstellung des Drucks im Drehrohr erfolgt zweckmäßig auf der Grundlage von Druckmessungen mittels eines Drucksensors. Als Drucksensor eignen sich z.B. solche vom Typ AMD 220, der Fa. Hartmann & Braun, die nach dem Druckmessumformer-(Membranmesswerk-)Prinzip arbeiten. Anwendungstechnisch zweckmäßig ist der Drucksensor (16) unmittelbar hinter dem Ausgang des Gasstroms aus dem Drehrohr positioniert. Ein weiterer Drucksensor kann sich vor dem Eingang des Gasstroms in das Drehrohr befinden. Dieser kann auch der einzige Drucksensor sein. Im Zusammenspiel von Drucksensor (16), Abgasverdichter (17) (Ventilator, der zum Regelventil hin gerichtet ansaugt), Kreisgasverdichter und Frischgaszufuhr kann dann die Druckeinstellung erfolgen. Werden die beiden Verdichter und die Frischgaszufuhr stationär betrieben (oder werden nicht regelbare Verdichter eingesetzt), ist das Regelventil (14) die alleinige Druckstellschraube.

Die Geschwindigkeit des durch das Drehrohr geführten Gasstroms liegt in typischer Weise bei Werten $\geq 0,1$ m/s und $\leq 2$ m/s.

Das Erhitzen des Materialguts im Drehrohr erfolgt erfindungsgemäß zweckmäßig durch Erhitzen des Drehrohs von außen. Prinzipiell kann dieses Erhitzen durch direkte Befeuerung erfolgen. D.h., unterhalb des Drehrohres befinden sich beispielsweise dort angebrachte Brenner, deren heiße Brenngase, beispielsweise unter der Mithilfe von Ventilatoren, um das von einer Einhüllenden umgebene Drehrohr geführt werden.

Bevorzugt ist das Drehrohr jedoch von einer Einhüllenden, z.B. einem Quader, umgeben, die auf ihrer Innenseite elektrisch beheizte (Widerstandsheizung) Elemente aufweist. Aus Gründen der Temperierhomogenität sollte die Einhüllende wenigstens auf zwei sich gegenüber liegenden Seiten solche Heizelemente aufweisen. Vorzugsweise umgeben die Heizelemente das Drehrohr jedoch allseitig. Ihr Abstand zur Drehrohraußenfläche beträgt typisch 10 bis 20 cm. In der hier beispielhaft beschriebenen Ausführungsform (Fig. 1) rotiert das Drehrohr frei in einem Quader (2), der vier in der Länge des Drehrohres aufeinanderfolgende, gleich lange, elektrisch beheizte (Widerstandsheizung) Heizzonen aufweist, von denen jede den Umfang des Drehrohrofens umschließt. Jede der Heizzonen kann den entsprechenden Drehrohrabschnitt auf Temperaturen zwischen Raumtemperatur und 850°C erhitzen. Die maximale Heizleistung jeder Heizzone beträgt 30 kW. Der Abstand zwischen elektrischer Heizzone und Drehrohraußenfläche beträgt etwa 10 cm. Am Anfang und am Ende ragt das Drehrohr ca. 30 cm aus dem Quader heraus.

Zusätzlich bietet ein Erhitzer (10) die Möglichkeit, den ins Drehrohr geführten Gasstrom vorab seines Eintritts ins Drehrohr auf die gewünschte Temperatur zu erwärmen (z.B. auf die für das Materialgut im Drehrohr gewünschte Temperatur), um so die Temperierung des im Drehrohrofen befindlichen Materialguts zu unterstützen. Geht mit der thermischen Behandlung der Vorläufermasse eine exotherme chemische Umsetzung in der Vorläufermasse einher, wird man den ins Drehrohr geführten Gasstrom erfindungsgemäß vorteilhaft mit einer Temperatur ins Drehrohr führen, die unterhalb der Temperatur liegt (z.B. bis zu 50°C, häufig bis zu 20°C), die für das Materialgut im Drehrohrofen vorgesehen ist. Geht mit der thermischen Behandlung der Vorläufermasse dagegen eine endotherme chemische Umsetzung in der Vorläufermasse einher, wird man den ins Drehrohr geführten Gasstrom erfindungsgemäß vorteilhaft mit einer Temperatur ins Drehrohr führen, die oberhalb der Temperatur liegt (z.B. bis zu 50°C, häufig bis zu 20°C), die für das Materialgut im Drehrohrofen vorgesehen ist.

Vom Prinzip her kann es sich beim Erhitzer (10) um einen Erhitzer jedweder Art handeln. Beispielsweise kann es sich um einen indirekten Wärmetauscher handeln. Solch ein Erhitzer kann prinzipiell auch als Kühler genutzt werden (durch Anpassung der Temperatur des Wärmeträgermediums, z.B. Einsatz von Kühlsohle). Der Erhitzer kann prinzipiell auch mittels direkter oder indirekter Befeuerung beheizt werden. Ebenso kommt eine direkte oder indirekte Dampfbeheizung in Betracht. Im Fall einer direkten Dampfbeheizung wird man in zweckmäßiger Weise die Kreisgasleitung wenigstens partiell begleitbeheizen, um ein unerwünschtes Kondensieren des im Gasstrom enthaltenen Wasserdampfes zu unterbinden bzw. um den Wasserdampf gegebenenfalls gezielt auszukondensieren. Häufig wird es sich um einen Elektroerhitzer handeln, bei dem der Gasstrom über Strom beheizte (Widerstandsheizung) Metalldrähte geführt wird. In der beispielhaft beschriebenen Ausführungsform wird ein CSN Durchlauferhitzer, Typ 97 D 80 der Fa. Schniewindt KG, 58805 Neuerade-DE eingesetzt, dessen maximale Leistung bei 1x50 kW + 1x30 kW liegt.

Sowohl die Beheizung des Drehrohrofens als auch des ins Drehrohr geführten Gasstroms wird in zweckmäßiger Weise über die Temperatur des Materialguts im Drehrohr gesteuert. Diese wird in der Regel über ins Materialgut ragende Thermoelemente bestimmt. In der beispielhaft beschriebenen Ausführungsform liegt dazu auf der zentralen Achse des Drehrohres eine Lanze (8), von der in Abständen von 800 mm insgesamt drei Thermoelemente (9) vertikal ins Materialgut führen. Als die Temperatur des Materialguts wird dann das arithmetische Mittel der drei Thermoelementtemperaturen verstanden. Innerhalb des im Drehrohr befindlichen Materialguts beträgt die maximale Abweichung zweier gemessener Temperaturen erfindungsgemäß zweckmäßig weniger als 30°C, bevorzugt weniger als 20°C, besonders bevorzugt weniger als 10°C und ganz besonders bevorzugt weniger als 5 bzw. 3°C.

Die Temperaturrate, mit der die im Drehrohr befindliche Vorläufermasse zum Zweck ihrer thermischen Behandlung aufgeheizt wird, liegt beim erfindungsgemäßen Verfahren normalerweise bei Werten $\leq 10°C/min$. Häufig beträgt die Temperaturrate $\leq 8°C/min$, vielfach $\leq 5°C/min$ oder $\leq 3°C/min$ und oft liegt sie bei Werten von $\leq 2°C/min$ oder $\leq 1°C/min$. In der Regel wird diese Temperaturrate jedoch $\geq 0,1°C/min$, meist $\geq 0,2°C/min$ und häufig $\geq 0,3°C/min$ oder $\geq 0,4°C/min$ betragen.

Wie bereits erwähnt, geht mit der thermischen Behandlung der Vorläufermasse einer katalytischen Aktivmasse häufig eine exotherme chemische Reaktion einher, an der vielfach auch Bestandteile des durch das Drehrohr geführten Gasstroms beteiligt sind. Für das erfindungsgemäße Verfahren wesentlich ist, dass diese chemische Reaktion nicht unkontrolliert verläuft, die durch die chemische Reaktion freigesetzte Wärme zügig abgeführt wird, da ein unkontrollierter Verlauf zu übermäßiger Wärmeentwicklung und unkontrollierter Temperaturerhöhung des thermisch behandelten Materialguts führen kann, was letztlich zu einer Minderung der Aktivmasseneigenschaften führt.

Um einen solch unkontrollierten Verlauf der thermischen Behandlung des Materialguts gegebenenfalls noch rechtzeitig abwehren zu können, verfügt eine für das erfindungsgemäße Verfahren geeignete Drehrohrvorrichtung in vorteilhafter Weise über eine Schnellkühlung. Diese kann z.B. in besonders effizienter Weise dadurch erfolgen, dass die das Drehrohr umgebende Einhüllende (der Quader in der beispielhaften Ausführungsform) auf einer Seite (z.B. im unteren Teil) Öffnungen (Längsausdehnung in typischer Weise 60 cm), Löcher, aufweist, durch die mittels eines Ventilators (5) (z.B. ein solcher vom Typ E 315/40-63, der Fa. Ventapp GmbH) Umgebungsluft oder vorab gekühlte Luft angesaugt und durch auf der anderen (gegenüberliegenden) Seite (z.B. im oberen Teil) der Einhüllenden befindliche Klappen (7) mit variabel einstellbarer Öffnung herausgeführt werden kann. Die Drehrohrbeheizung wird dabei häufig gleichzeitig abgeschaltet. Eine solche Schnellkühlung ermöglicht es auch, die thermische Behandlung punktgerecht zu beenden und so eine zu weitgehende thermische Behandlung zu verhindern. Als zusätzliche Maßnahme kann der Gasstrom gekühlt (im "Erhitzer (10)") ins Drehrohr geführt werden.

Die Schnellkühlung gestattet es, z.B. am Ende der thermischen Behandlung der Vorläufermasse, die Temperatur des im Drehrohrofen befindlichen Materialguts innerhalb eines Zeitraums von $\leq 5$ h, meist $\leq 4$ h, häufig $\leq 2$ h, um wenigstens 300°C zu senken. In der Regel wird dieser Abkühlzeitraum aber nicht weniger als 0,5 h betragen.

Zwischen dem Druckminderer (15) und dem Erhitzer (10) wird man zum tatsächlich rezirkulierten Kreisgasanteil (19) anwendungstechnisch zweckmäßig die Frischgasdosierung vornehmen. Wie in der in Figur 1 dargestellten beispielhaften Ausführungsform wird häufig eine Frischgasgrundlast (z.B. Stickstoff (20)) zudosiert. Mit wenigstens einem Splitter kann dann auf die Messwerte der verschiedenen angewandten Bestandteilssensoren reagiert und die Bestandteilsgehalte des im dem Drehrohr zugeführten Gasstrom im Detail geregelt werden. In der beispielhaften Ausführungsform ist dies ein Stickstoff/Luft Splitter (21), der auf den Messwert des Sauerstoffsensors reagiert.

Die Bestandteilsensoren sind in zweckmäßiger Weise vor dem Kreisgasverdichter angebracht (18). Sie könnten jedoch auch an anderer Stelle angebracht sein.

Mit diesen Sensoren werden häufig reduzierend wirkende Gasbestandteile wie $NH_3$, CO, NO, $N_2O$, Essigsäure, Aerosole (z.B. Ammoniumacetat) und oxidierend wirkende Bestandteile wie $NO_2$ und $O_2$ bestimmt, deren Gehalt sich häufig in besonders engen Grenzen bewegen muß.

In der beispielhaften Ausführungsform sind zwei Sensoren (18) angebracht, nämlich einer zur Bestimmung des Ammoniakgehaltes und einer zur Bestimmung des Sauerstoffgehaltes. Der Ammoniaksensor arbeitet generell bevorzugt nach einem optischen Messprinzip (die Absorption von Licht bestimmter Wellenlänge korreliert proportional. zum Ammoniakgehalt des Gases). In der beispielhaften Ausführungsform ist es ein Gerät der Fa. Perkin & Elmer vom Typ MCS 100. Sauerstoffsensoren fußen dagegen in der Regel auf den paramagnetischen Eigenschaften des Sauerstoffs. In der beispielhaften Ausführungsform wird als Sauerstoffsensor in zweckmäßiger Weise ein Oximat der Fa. Siemens vom Typ Oxymat MAT SF 7MB 1010-2CA01-1AA1-Z verwendet.

Im Fall von katalytischen Multimetalloxidaktivmassen erfolgt beim Übergang von der Vorläufermasse zur katalytischen Aktivmasse im Rahmen der erfindungsgemäßen thermischen Behandlung häufig eine Sauerstoffaufnahme aus der im Drehrohr herrschenden Gasatmosphäre. Mittels vor dem Gasstromeingang in das Drehrohr und hinter dem Gasstromausgang aus dem Drehrohr angebrachten Sauerstoffsensoren kann diese Sauerstoffaufnahme online quantitativ detektiert und das Sauerstoffangebot im das Drehrohr durchströmenden Gasstrom entsprechend angepasst werden (erfolgt eine solche Anpassung nicht, wird normalerweise das Sauerstoffangebot im dem Drehrohr zugeführten Gasstrom im wesentlichen konstant gehalten). Über- oder Unteroxidationen der Aktivmasse lassen sich so vermeiden und dadurch die Katalysatorperformance verbessern. Völlig analog kann auch mit reduzierend wirkenden Gasbestandteilen vorgegangen werden.

Die erfindungsgemäße Kreisgasfahrweise erweist sich in diesem Zusammenhang auch dann als von besonderem Vorteil, wenn im Verlauf der thermischen Behandlung der Charakter der im Drehrohrofen herrschenden Gasatmosphäre von "reduzierend" nach "oxidierend" oder umgekehrt gewechselt wird. Gestattet sie es doch im einfacher Weise diesen Wechsel so durchzuführen, dass sich die Gasatmosphäre im Drehrohr stets außerhalb des Explosionsbereichs befindet.

In Testversuchen des erfindungsgemäßen Verfahrens hat sich häufig gezeigt, dass die Gasbestandteilssensoren falsche Werte angezeigt haben.

Genaueste Untersuchungen dieses Sachverhalts haben ergeben, dass dies darauf zurückzuführen ist, dass der durch das Drehrohr strömende Gasstrom in der Regel von der thermisch zu behandelnden Vorläufermasse herrührenden Feinststaub mit austrägt, der sich auf die Sensorenoberfläche (die Sensoren arbeiten in der Regel so, dass sie aus dem Gasstrom Teilmengen ansaugen) legt und die Messungen verfälscht. Die alleinige Anwendung von vor den Sensoren angebrachten Filtern konnten diesem Problem nicht abhelfen, da sie in Abhängigkeit von der Zeit verstopfen und so die Meßergebnisse gleichfalls verfälschen.

Als Problemlösung eignet sich ein hinter dem Austritt (aber vor den Sensoren) des durch das Drehrohr geführten Gasstroms angebrachter Zyklon (12), zur Abtrennung von mit dem Gasstrom mitgerissenen Feststoffpartikeln (der Zentrifugalabscheider trennt in der Gasphase suspendierte Feststoffpartikel durch Zusammenwirken von Zentrifugal- und Schwerkraft ab; die Zentrifugalkraft des als Spiralwirbel rotierenden Gasstroms beschleunigt die Sedimentation der suspendierten Teilchen).

Die Mitverwendung eines solchen Zyklons verhindert auch, dass sich der Feinststaub auf den Wänden der Kreisgasleitung niederschlägt (ein solcher Feinststaubniederschlag würde sich beispielsweise im Fall eines Produktwechsels nachteilig bemerkbar machen; d.h., wird die thermisch zu behandelnde Vorläufermasse gewechselt, wird die Nachfolgemasse im Rahmen einer Kreisgasführung gegebenenfalls mit dem an den Wandungen niedergeschlagenen Staub der zuvor behandelten Vorläufermasse kontaminiert; auch kann der Feinststaub den Erhitzer (10) beschädigen (Veränderung des Wärmeübergangs) oder verstopfen).

Der im Zyklon abgeschiedene Feststoff wird entsorgt.

Der durch den Zyklon bewirkte Druckverlust wird durch den Kreisgasverdichter ausgeglichen.

Anstelle eines Zyklons kann prinzipiell auch jede andere Vorrichtung eingesetzt werden, die dazu geeignet ist, feinteilige Feststoffe aus einer gasförmigen Dispersionsphase abzutrennen.

Der ausgeschleuste Kreisgasanteil (22) (Abgas) enthält häufig nicht völlig unbedenkliche Gase wie $NO_x$, CO, Essigsäure, $NH_3$, usw., weshalb diese im Normalfall in einer Abgasreinigungsvorrichtung abgetrennt werden (23).

Dazu wird das Abgas in der Regel zunächst über eine Waschkolonne geführt (dies ist im wesentlichen eine an Einbauten freie Kolonne, die vor ihrem Ausgang eine trennwirksame Packung enthält; das Abgas und wässriger Sprühnebel werden im Gleichstrom oder im Gegenstrom geführt (2 Sprühdüsen mit entgegengesetzter Sprührichtung).

Aus der Waschkolonne kommend wird das Abgas zweckmäßig in eine Vorrichtung geführt, die einen Feinstaubfilter (in der Regel ein Bündel aus Schlauchfiltern) enthält, aus dessen Innerem das eingedrungene Abgas weggeführt wird. Dann wird abschließend vorteilhaft in einer Muffel verbrannt.

Zur Regelung der Menge des vom Sperrgas verschiedenen, dem Drehrohr insgesamt zugeführten Gasstroms, eignet sich beim erfindungsgemäßen Verfahren vor allem das Prinzip der Massendurchflussmessung auf der Grundlage von Corioliskräften, das Prinzip der Blendenmessung auf der Grundlage von Druckdifferenzen und das Prinzip der Thermalkonvektion.

In der beispielhaften Ausführungsform erfolgt die Messung der Menge des vom Sperrgas verschiedenen, dem Drehrohr zugeführten, Gasstroms mittels eines Sensors (28) der Fa. KURZ Instruments, Inc., Montery (USA) vom Typ Modell 455 Jr (Messprinzip: thermalkonvektive Massendurchflussmessung unter Anwendung eines Gleichtemperatur-Anemometers).

Die beschriebene Drehrohrofenvorrichtung kann sowohl kontinuierlich als auch diskontinuierlich betrieben werden. Erfindungsgemäß bevorzugt wird sie diskontinuierlich betrieben. Materialgut und Gasphase werden im kontinuierlichen Betrieb vorzugsweise im Gegenstrom durch den Drehrohrofen geführt. Beispielsweise die Multimetalloxidmassen der DE-A 19855913 bzw. WO 02/24620 sowie die zu deren Herstellung benötigten vorgebildeten Phasen (z.B. die Bismutwolframate und die Bismutmolybdate) sind durch kontinuierliche thermische Behandlung der Vorläufermasse im Drehrohrofen in hervorragender Weise erhältlich.

Zur Durchführung des erfindungsgemäßen Verfahrens eignen sich demnach vor allem Drehrohrofenvorrichtungen, die umfassen:

a)   wenigstens einen Kreisgasverdichter (13);

b)   wenigstens einen Abgasverdichter (17);

c)   wenigstens einen Druckminderer (15);

d)   wenigstens eine Frischgaszufuhr ((20), (21));

e)   wenigstens ein beheizbares Drehrohr (1); und

f)   wenigstens eine Kreisgasleitung.

Werden Verdichter (13) und (17) angewendet, die nicht regelbar sind, wird die Drehrohrofenvorrichtung noch wenigstens ein Regelventil (14) umfassen.

Erfindungsgemäß vorteilhafte Ausführungsformen umfassen darüber hinaus noch wenigstens einen Erhitzer (10).

Ganz besonders bevorzugte Ausführungsformen enthalten zusätzlich wenigstens einen Zyklon integriert.

Darüber hinaus ist es von Vorteil, wenn die Drehrohrofenvorrichtung eine Vorrichtung zur Schnellkühlung aufweist.

Ist beabsichtigt, die Drehrohrofenvorrichtung auch für thermische Behandlungen von Materialgut einzusetzen, bei denen der durch das Drehrohr geführte Gasstrom auch nicht wenigstens anteilig im Kreis geführt wird, ist es zweckmäßig, wenn die Verbindung zwischen Zyklon (12) und Kreisgasverdichter (13) nach dem Dreiwegehahnprinzip (26) geschlossen und der Gasstrom auf direktem Weg in die Abgasreinigungsvorrichtung (23) geführt werden kann. Die hinter dem Kreisgasverdichter befindliche Verbindung zur Abgasreinigungsvorrichtung wird in diesem Fall ebenfalls nach dem Dreiwegehahnprinzip geschlossen. Besteht der Gasstrom im wesentlichen aus Luft, wird diese in diesem Fall über den Kreisgasverdichter (13) angesaugt (27). Die Verbindung zum Zyklon wird ebenfalls nach dem Dreiwegehahnprinzip geschlossen. Vorzugsweise wird der Gasstrom in diesem Fall durch das Drehrohr gesaugt, so dass der Drehrohrinnendruck kleiner als der Umgebungsdruck ist.

Bevorzugt erfolgt vorstehende Behandlung kontinuierlich und im Gegenstrom von Materialgut und Gasstrom. D.h., das thermisch zu behandelnde Materialgut wird am entgegengesetzten Ende wie der Gasstrom (am höchstgelegenen Punkt des Drehrohrs) in den Drehrohrofen eingetragen, und am entgegengesetzten Ende wie der Gasstrom aus dem Drehrohrofen ausgetragen.

Bei der beispielhaften Ausführungsform der Drehrohrvorrichtung wird der Druck hinter dem Drehrohrausgang (des Gasstroms) bei kontinuierlichem Betrieb vorteilhaft -0,8 mbar unterhalb des Außendrucks liegend eingestellt. Bei diskontinuierlichem Betrieb beträgt die gleiche Druckeinstellung -0,2 mbar.

Wie bereits erwähnt, eignet sich das erfindungsgemäße Verfahren zur thermischen Behandlung von Vorläufermassen von katalytischen Aktivmassen aller Art. Dabei kann die Vorläufermasse sowohl in Pulverform als auch zu speziellen (Katalysator) Formkörpern geformt der erfindungsgemäßen thermischen Behandlung unterworfen werden.

Es eignet sich zur Herstellung von katalytisch aktiven Multielementoxidmassen, die wenigstens eines der Elemente Nb und W sowie die Elemente Mo und V enthalten, wobei der molare Anteil des Elementes Mo an der Gesamtmenge aller von Sauerstoff verschiedenen Elemente der katalytisch aktiven Multielementoxidmasse 20 mol-% bis 80 mol-% beträgt, das molare Verhältnis von in der katalytisch aktiven Multielementoxidmasse enthaltenem Mo zu in der katalytisch aktiven Multielementoxidmasse enthaltenem V, Mo/V, 15:1 bis 1:1 beträgt, und das entsprechende molare Verhältnis Mo/(Gesamtmenge aus W und Nb) 80:1 bis 1:4 beträgt. Häufig enthalten solche Multielementoxidmassen noch Cu im entsprechenden molaren Verhältnis Mo/Cu von 30:1 bis 1:3. Nachfolgend sollen die verschiedenen Vorteile des erfindungsgemäßen Verfahrens an Beispielen solcher Multielementoxidmassen nochmals aufgezeigt werden. Sie sind z.B. dadurch erhältlich, dass man aus Ausgangsverbindungen, die die von Sauerstoff verschiedenen elementaren Konstituenten der Multielementoxidmasse als Bestandteile enthalten, ein inniges, auch Ammoniumionen enthaltendes, Trockengemisch (die Vorläufermasse) herstellt und dieses bei Temperaturen im Bereich von 300 bis 450°C (Materialguttemperatur) in einer $O_2$ und $NH_3$ aufweisenden Gasatmosphäre thermisch behandelt. Dabei ist die Gasatmosphäre, in der die thermische Behandlung erfolgen sollte, gemäß der Lehre der EP-B 72448 z.B. eine solche, die

- zu jedem Zeitpunkt der thermischen Behandlung 0,5 bis 4 Vol.-% $O_2$,
- über die Gesamtdauer der reduktiven thermischen Behandlung gemittelt 1 bis 8 Vol.-% $NH_3$, sowie
- Wasserdampf und/oder Inertgas als Restmengen enthält,

wobei der $NH_3$-Gehalt der Atmosphäre während der thermischen Behandlung ein Maximum durchläuft, das unterhalb von 20 Vol.-% liegt.

Vorgenannte Multielementoxidmassen können neben den Elementen Nb und/oder W, sowie Mo, V und gegebenenfalls Cu zusätzlich z.B. die Elemente Ta, Cr, Ce, Ni, Co, Fe, Mn, Zn, Sb, Bi, Alkali (Li, Na, K, Rb, Cs), H, Erdalkali (Mg, Ca, Sr, Ba), Si, Al, Ti und Zr enthalten. Natürlich kann die Multielementoxidaktivmasse aber auch nur aus den Elementen Nb und/oder W sowie Mo, V und gegebenenfalls Cu bestehen. Sie eignen sich insbesondere als Aktivmassen für Katalysatoren zur heterogen katalysierten partiellen Gasphasenoxidation von Acrolein zu Acrylsäure.

Als Aktivmasse für Katalysatoren zur heterogen katalysierten partiellen Gasphasenoxidation von Acrolein zu Acrylsäure ganz besonders geeignete katalytisch aktive Multielementoxidmassen genügen dabei der nachfolgenden allgemeinen Stöchiometrie I

$$Mo_{12}V_aX^1_bX^2_cX^3_dX^4_eX^5_fX^6_gO_n \qquad (I),$$

in der die Variablen folgende Bedeutung haben:

$X^1$ = W, Nb, Ta, Cr und/oder Ce,
$X^2$ = Cu, Ni, Co, Fe, Mn und/oder Zn,
$X^3$ = Sb und/oder Bi,
$X^4$ = einer oder mehrere Alkalimetalle (Li, Na, K, Rb, Cs) und/oder H,
$X^5$ = eines oder mehrere Erdalkalimetalle (Mg, Ca, Sr, Ba),
$X^6$ = Si, Al, Ti und/oder Zr,
a = 1 bis 6,
b = 0,2 bis 4,
c = 0 bis 18, vorzugsweise 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in I bestimmt wird, und

wobei die Variablen innerhalb der vorgegebenen Bereiche mit der Maßgabe auszuwählen sind, dass der molare Anteil des Elementes Mo an der Gesamtmenge aller von Sauerstoff verschiedenen Elemente der Multielementoxidmasse (I) 20 mol-% bis 80 mol% beträgt, das molare Verhältnis von in der katalytisch aktiven Multielementoxidmasse (I) enthaltenem Mo zu in der katalytisch aktiven Multielementoxidmasse (I) enthaltenem V, Mo/V, 15:1 bis 1:1 beträgt, und das entsprechende molare Verhältnis Mo/(Gesamtmenge aus W und Nb) 80:1 bis 1:4 beträgt (und das entsprechende molare Verhältnis Mo/Cu 30:1 bis 1:3 beträgt, falls die Multielementoxidmasse Cu enthält).

Unter den aktiven Multielementoxidmassen (I) sind wiederum jene bevorzugt, bei denen die Variablen in den folgenden Bereichen liegen:

$X^1$ = W, Nb und/oder Cr,
$X^2$ = Cu, Ni, Co und/oder Fe,
$X^3$ = Sb,
$X^4$ = Na und/oder K,
$X^5$ = Ca, Sr und/oder Ba,
$X^6$ = Si, Al und/oder Ti,
a = 2,5 bis 5,
b = 0,5 bis 2,
c = 0,5 bis 3,
d = 0 bis 2,
e = 0 bis 0,2,
f = 0 bis 1,
g = 0 bis 15 und
n = eine Zahl, die durch die Wertigkeit und Häufigkeit der von Sauerstoff verschiedenen Elemente in I bestimmt wird.

Ganz besonders bevorzugt sind jedoch die nachfolgenden Multielementoxidaktivmassen II unmittelbare Verfahrensprodukte des erfindungsgemäßen Verfahrens:

$$Mo_{12}V_aX^1{}_bX^2{}_cX^5{}_fX^6gO_n \qquad (II),$$

in der die Variablen folgende Bedeutung haben:

$X^1$ = W und/oder Nb,
$X^2$ = Cu und/oder Ni,
$X^5$ = Co und/oder Sr,
$X^6$ = Si und/oder Al,
a = 3 bis 4,5,
b = 1 bis 1,5,
c = 0,75 bis 2,5,
f = 0 bis 0,5,
g = 0 bis 8 und
n = eine Zahl, die durch die Wertigkeit und Häufigkeit der von Sauerstoff verschiedenen Elemente in II bestimmt wird, und

wobei die Variablen innerhalb der vorgegebenen Bereiche mit der Maßgabe auszuwählen sind, dass der molare Anteil des Elementes Mo an der Gesamtmenge aller von Sauerstoff verschiedenen Elemente der Multielementoxidaktivmasse (II) 20 mol-% bis 80 mol-% beträgt, das molare Verhältnis von in der katalytisch aktiven Multielementoxidmasse (II) enthaltenem Mo zu in der katalytisch aktiven Multielementoxidmasse (II) enthaltenem V, Mo/V, 15:1 bis 1:1 beträgt, das entsprechende molare Verhältnis Mo/Cu 30:1 bis 1:3 und das entsprechende molare Verhältnis Mo/(Gesamtmenge aus W und Nb) 80:1 bis 1:4 beträgt.

Zur Herstellung von solchen Multielementoxidmassen geht man wie bereits gesagt von in an sich bekannter Weise geeigneten Quellen (Ausgangsverbindungen) der von Sauerstoff verschiedenen elementaren Konstituenten der gewünschten Multielementoxidaktivmasse in im der Multielementoxidaktivmasse angestrebten jeweiligen stöchiometrischen Verhältnis aus, und erzeugt aus diesen ein möglichst inniges, vorzugsweise feinteiliges, Trockengemisch, welches dann der thermischen Behandlung unterworfen wird, wobei die thermische Behandlung vor oder nach der Formung zu (Katalysator)Formkörpern bestimmter Geometrie erfolgen kann. Erfindungsgemäß vorteilhaft erfolgt sie davor. Dabei kann es sich bei den Quellen entweder bereits um Oxide handeln, oder um solche Verbindungen, die durch Erhitzen, wenigstens in Anwesenheit von Sauerstoff, in Oxide überführbar sind. Neben den Oxiden kommen daher als Ausgangsverbindungen vor allem Halogenide, Nitrate, Formiate, Oxalate, Acetate, Carbonate oder Hydroxide in Betracht.

Geeignete Ausgangsverbindungen des Mo, V, W und Nb sind auch deren Oxoverbindungen (Molybdate, Vanadate, Wolframate und Niobate) bzw. die von diesen abgeleiteten Säuren. Sauerstoff haltige Quellen sind ebenfalls günstig.

Der erforderliche Gehalt des innigen Trockengemischs an Ammoniumionen kann in einfacher Weise dadurch realisiert werden, dass man in das innige Trockengemisch eine entsprechende Menge Ammoniumionen einarbeitet. Zweckmäßigerweise lassen sich die Ammoniumionen in das innige Trockengemisch z.B. dadurch einbringen, dass man als Quellen der Elemente Mo, V, W oder Nb die entsprechenden Ammoniumoxometallate verwendet. Beispiele hierfür sind Ammoniummetaniobat, Ammoniummetavanadat, Ammoniumheptamolybdattetrahydrat und Ammoniumparawolframatheptahydrat. Selbstverständlich können in das thermisch zu behandelnde innige Trockengemisch aber auch unabhängig von den als Quellen der Multielementoxidaktivmassenkonstituenten erforderlichen Ausgangsverbindungen Ammoniumlieferanten wie $NH_4NO_3$, oder $NH_4Cl$, oder Ammoniumacetat, oder Ammoniumcarbonat, oder Ammoniumhydrogencarbonat, oder $NH_4OH$, oder $NH_4CHO_2$, oder Ammoniumoxalat eingearbeitet werden.

Das innige Vermischen der Ausgangsverbindungen kann prinzipiell in trockener oder in nasser Form erfolgen. Erfolgt es in trockener Form, werden die Ausgangsverbindungen zweckmäßigerweise als feinteilige Pulver eingesetzt und nach dem Mischen z.B. zu (Katalysator)Formkörpern gewünschter Geometrie verpresst (z.B. tablettiert), die dann der erfindungsgemäßen thermischen Behandlung unterworfen werden.

Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen und Ausgangsverbindungen ausgegangen wird. Als Lösungsmittel wird bevorzugt Wasser eingesetzt. Anschließend wird die wässrige Masse (Lösung oder Suspension) getrocknet und das so erhaltene innige Trockengemisch gegebenenfalls unmittelbar thermisch behandelt. Vorzugsweise erfolgt der Trocknungsprozess durch Sprühtrocknung (die Austrittstemperaturen betragen in der Regel 100 bis 150°C) und unmittelbar im Anschluss an die Fertigstellung der wässrigen Lösung oder Suspension. Das dabei anfallende Pulver kann unmittelbar durch Pressen geformt werden. Häufig erweist es sich jedoch für eine unmittelbare Weiterverarbeitung als zu feinteilig, weshalb es dann unter Zusatz von z.B. Wasser zweckmäßigerweise geknetet wird. Vielfach erweist sich beim Kneten ein Zusatz einer niederen organischen Carbonsäure (z.B. Essigsäure) als vorteilhaft (typische Zusatzmengen liegen bei 5 bis 10 Gew.-% bezogen auf eingesetzte Pulvermasse).

Die anfallende Knetmasse wird anschließend entweder zur gewünschten Geometrie geformt, getrocknet und dann der thermischen Behandlung unterworfen (führt zu sogenannten Vollkatalysatoren) oder nur zu Stränglingen geformt, diese thermisch behandelt und danach zu einem Pulver vermahlen (üblicherweise < 80 μm) welches normalerweise unter Zusatz einer geringen Menge Wasser sowie gegebenenfalls weiterer üblicher Bindemittel als Feuchtmasse auf inerte Trägerkörper aufgetragen wird. Nach beendeter Beschichtung wird nochmals getrocknet und so ein einsatzfähiger Schalenkatalysator erhalten. Erfolgt das innige Vermischen der Ausgangsverbindungen in Form einer z.B. wässrigen Lösung, so können mit selbiger auch inerte poröse Trägerkörper getränkt, getrocknet und anschließend zu Trägerkatalysatoren erfindungsgemäß thermisch behandelt werden. Bei der Herstellung von Schalenkatalysatoren kann das Beschichten der Trägerkörper auch vorab der thermischen Behandlung, d.h. z.B. mit dem befeuchteten Sprühpulver, erfolgen.

Für Schalenkatalysatoren geeignete Trägermaterialien sind z.B. poröse oder unporöse Aluminiumoxide, Siliciumdioxid, Thoriumdioxid, Zirkondioxid, Siliciumcarbid oder Silikate wie Magnesium- oder Aluminiumsilicat (z.B. Steatit des Typs C 220 der Fa. CeramTec).

Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder mit Splittauflage, bevorzugt werden.

Geeignet ist die Verwendung von im wesentlichen unporösen, oberflächenrauhen, kugelförmigen Trägern aus Steatit (z.B. Steatit des Typs C 220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper.

Die Beschichtung der Trägerkörper mit feinteiliger wie beschrieben durch thermische Behandlung erhältlicher Multielementoxidaktivmasse bzw. deren noch thermisch zu behandelnder feinteiliger Vorlaufsmasse (innigesTrockengemisch) wird in der Regel in einem drehbaren Behälter ausgeführt, wie es z.B. aus der DE-A 2909671, der EP-A 293859 oder aus der EP-A 714700 bekannt ist. Die Verfahrensweise der EP-A 714700 ist dabei bevorzugt.

Zweckmäßigerweise wird zur Beschichtung der Trägerkörper mit der aufzubringenden Pulvermasse der Trägerkörper befeuchtet. Nach dem Aufbringen wird normalerweise mittels heißer Luft getrocknet. Die Schichtdicke der auf den Träger aufgebrachten Pulvermasse wird zweckmäßigerweise im Bereich 10 bis 1000 $\mu$m, bevorzugt im Bereich 50 bis 500 $\mu$m und besonders bevorzugt im Bereich 150 bis 250 $\mu$m liegend, gewählt.

Im Fall von Vollkatalysatoren kann die Formgebung, wie bereits erwähnt, ebenfalls vor oder nach der Durchführung der thermischen Behandlung erfolgen.

Beispielsweise können aus der Pulverform der erfindungsgemäß erhältlichen Multielementoxidaktivmasse oder ihrer noch nicht thermisch behandelten Vorläufermasse (dem innigen Trockengemisch) durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich können die relevanten Multielementoxidaktivmassen auch in Pulverform, d.h. nicht zu bestimmten Katalysatorgeometrien geformt, als Katalysatoren für die heterogen katalysierte Partialoxidation von Acrolein zu Acrylsäure eingesetzt werden (z.B. auch im Wirbelbett).

Die partielle Gasphasenoxidation von Acrolein zu Acrylsäure selbst kann mit den beschriebenen Multielementoxidaktivmassen z.B. wie in der EP-A 724481, oder wie in der DE-A 19910508 beschrieben durchgeführt werden.

Hinsichtlich der thermischen Behandlung der Vorläufermassen der vorstehend beschriebenen Multielementoxidmassen stellt die EP-B 724481 nun fest (S.5, Zeilen 25 ff):

"Die erfindungsgemäß erforderliche Calcinationsatmosphäre lässt sich in einfacher Weise beispielsweise dadurch realisieren, dass man in einem Ofen calciniert, durch den man ein Gasgemisch führt, das die entsprechende Zusammensetzung bezüglich $O_2$, $NH_3$, und Inertgasen/Wasserdampf aufweist. In einer weniger bevorzugten Ausführungsform kann der erforderlich mittlere Ammoniakgehalt der Calcinierungsatmosphäre auch dadurch realisiert werden, dass man in die zu calcinierende Trockenmasse eine entsprechende Menge Ammoniumionen einarbeitet, welche sich im Verlauf der Calcinierung unter $NH_3$-Abgabe zersetzen."

Durchgeführt wurde die Calcinierung in der EP-B 724481 in Umluftöfen, wobei diesen u.a. ein Gasgemisch zugeführt wurde, das einen bestimmten Volumenanteil an $NH_3$ (Vol.-%) enthielt.

Mit der dieser Anmeldung zugrunde liegenden Erfindung wird nun aus der weniger bevorzugten Ausführungsform der EP-B 724481 eine bevorzugte Ausführungsform. Dies ist unter anderem darauf zurückzuführen, dass das erfindungsgemäße Verfahren einer externen Zugabe von $NH_3$ in die Calcinationsatmosphäre nicht mehr bedarf, sondern durch die Kreisgasführung mit den in der thermisch zu behandelnden Vorläufermasse enthaltenen Ammoniumionen als Ammoniakquelle auskommt. Als weiterer Vorteil senkt das Verfahren der vorliegenden Patentanmeldung durch die Kreisgasführung den Energieaufwand für die thermische Behandlung, da sie den im Kreisgas enthaltenen Energieinhalt weiternutzt. Außerdem erlaubt das erfindungsgemäße Verfahren infolge der sich im Drehrohr stetig selbst homogenisierenden Schüttung die Herstellung großer Mengen an katalytischen Aktivmassen (insbesondere katalytisch aktiven Multielementoxidmassen) mit besonders enger Aktivitätsverteilung innerhalb der hergestellten Charge. Dies ist vor allem bei den besagten katalytisch aktiven Multielementoxidmassen, die wenigsten eins der Elemente Nb und W sowie die Elemente Mo, V und Cu enthalten und die zur Herstellung von Katalysatoren für die heterogen katalysierte partielle Gasphasenoxidation von Acrolein zu Acrylsäure verwendet werden sollen, von Bedeutung. Vor allem dann, wenn dies Gasphasenpartialoxidation bei hohen Acroleinlasten durchgeführt wird, wie es z.B. die DE-A 10307983, die DE-A 19948523 und die DE-A 19910508 beschreiben.

In der Regel wird diese Gasphasenpartialoxidation des Acroleins nämlich in einem eine oder mehrere Temperaturzonen aufweisenden Rohrbündelreaktor durchgeführt wie sie z.B. die EP-A 700714, die EP-A 700 893, die DE-A 19910508, die DE-A 19948523, die DE-A 19910506, die DE-A 19948241, die DE-C 2830765, die DE-C 2513405, die US-A 3147084, die DE-A 2201528, die EP-A 383224 und die DE-A 2903218 beschreiben.

Die Feststoffkatalysatorschüttung befindet sich in den Metallrohren (Katalysatoren) des Rohrbündelreaktors und um die Metallrohre werden das oder die Temperiermedien geführt (bei mehr als einer Temperaturzone wird eine entsprechende Anzahl räumlich getrennter Temperiermedien um die Metallrohre geführt). Das Temperiermedium ist in der Regel eine Salzschmelze. Durch die Kontaktrohre wird das Reaktionsgemisch geführt.

Die Kontaktrohre sind üblicherweise aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 2 bis 4 m.

Anwendungstechnisch zweckmäßig beläuft sich die im Rohrbündelbehälter untergebrachte Anzahl an Kontaktrohren auf wenigstens 5000, vorzugsweise auf wenigstens 10 000. Häufig beträgt die Anzahl der im Reaktorbehälter untergebrachten Kontaktrohre 15 000 bis 30 000. Rohrbündelreaktoren mit einer oberhalb von 40 000 liegen Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290).

Als Wärmeaustauschmittel besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

Obwohl durch geeignete Strömungsverhältnisse in den Rohrbündelreaktoren generell zu erreichen versucht wird, dass über den Reaktorschnitt betrachtet auf jedes einzelne Kontaktrohr die gleiche Salzbadtemperatur einwirkt, kann das Vorliegen von Temperaturgradienten über den Reaktorquerschnitt in der Praxis nicht völlig vermieden werden. Wenn nun zusätzlich zu den Temperaturgradienten über den Reaktorquerschnitt ausgeprägte Aktivitätsgradienten über die individuellen Kontaktrohrbeschickungen kommen, so kann dies die Sicherheit des Betriebs des Rohrbündelreaktors beeinträchtigen, da die Wärmeentwicklung aufgrund der exothermen heterogen katalysierten Festbettpartialoxidation von Acrolein zu Acrylsäure in den individuellen Kontaktrohren eines Rohrbündelreaktors in einem solchen Fall signifikant unterschiedlich wäre. Letzteres deshalb, weil eine erhöhte Aktivität der Kontaktrohrbeschickung bedeutet, dass bei gleicher Temperatur pro Zeiteinheit mehr umgesetzt und damit mehr Wärme entwickelt wird.

Das erfindungsgemäße Verfahren gestattet nun eine regelmäßige Beschickung von Rohrbündelreaktoren (mit Katalysatoren die als Aktivmasse die besagten katalytisch aktiven Multielementoxidmassen, die wenigstens eines der Elemente Nb und W sowie die Elemente Mo, V und gegebenenfalls Cu enthalten) mit 5 000 bis 40 000 Kontaktrohren, die so beschaffen ist, dass bei einer willkürlich herausgegriffenen Stichprobe von 12 Kontaktrohren der Unterschied zwischen der arithmetisch mittleren Aktivität und der höchsten bzw. kleinsten Aktivität nicht mehr als 8°C, häufig nicht mehr als 6°C, vielfach nicht mehr als 4°C und in günstigen Fällen nicht mehr als 2°C beträgt.

Bemerkenswert am erfindungsgemäßen Verfahren ist, dass vorstehendes Ergebnis auch erreicht wird, wenn die im Rohrbündelreaktor insgesamt enthaltene Aktivmasse in weniger als 100, bzw. weniger als 75, bzw. weniger als 50 Ansätzen der thermischen Behandlung einer Vorläufermasse hergestellt wurde. Häufig beträgt die Anzahl dieser Ansätze 5 bis 40.

Als Maß für die Aktivität der Kontaktrohrbeschickung wird dabei die Temperatur verwendet, die ein das einzelne Kontaktrohr umspülende Salzbad (Gemisch aus 53 Gew.-% Kaliumnitrat, 40 Gew.-% Natriumnitrit und 7 Gew.-% Natriumnitrat) aufweisen muß, damit bei einmaligem Durchgang eines Reaktionsgasgemisches aus 4,8 Vol.-% Acrolein, 7 Vol.-% Sauerstoff, 10 Vol.% Wasserdampf und 78,2 Vol.-% Stickstoff (bei einer Belastung der Katalysatorbeschickung mit 85 Nl Acrolein/l Katalysatorbeschickung x h) durch das beschickte Katalysatorrohr ein Acroleinumsatz von 97 mol.-% erzielt wird (unter "l Katalysatorbeschickung" werden dabei die Volumina innerhalb des Katalysatorrohres nicht mit einbezogen, wo sich reine Vor- oder Nachschüttungen aus Inertmaterial befinden, sondern nur die Schüttvolumina, die Katalysatorformkörper (gegebenenfalls mit Inertmaterial verdünnt enthalten).

Eine wie beschrieben erzielbare Beschickung von Kontaktrohren in Rohrbündelreaktoren ist vor allem dann wichtig, wenn der Rohrbündelreaktor bei einer Acrolein-Belastung der Katalysatorbeschickung betrieben wird, die ≥135 Nl/l·h, oder ≥150 Nl/l·h, oder ≥160 Nl/l·h, oder ≥170 Nl/l·h, oder ≥180 Nl/l·h, oder ≥200 Nl/l·h, oder ≥220 Nl/l·h, oder ≥240 Nl/l·h beträgt. Selbstredend ist eine solche Katalysatorbeschickung auch bei kleineren Acroleinbelastungen vorteilhaft.

In der Regel wird die Acroleinbelastung der Katalysatorbeschichtung jedoch ≤ 350 Nl/l·h, oder ≤ 300 Nl/l·h, oder ≤ 250 Nl/l·h betragen.

Im übrigen kann der Rohrbündelreaktor mit der Partialoxidation von Acrolein zu Acrylsäure so betrieben werden, wie es die DE-A 10307983, die DE-A 19948523 und die DE-A 19910508 beschreiben.

Abschließend sei noch festgehalten, dass mit dem erfindungsgemäßen Verfahren auf einfache Weise Verweilzeiten der thermisch zu behandelnden Vorläufermasse im Drehrohrofen von ≥ 5 h, bzw. ≥ 10 h, oder ≥ 15 h, oder ≥ 20 h, oder ≥ 25 h möglich sind. In der Regel wird diese Verweilzeit ≤ 50 h betragen.

Das erfindungsgemäße Verfahren einet sich zur Herstellung von Multielementoxidaktivmassen die die Elemente Mo, V, wenigstens eines der beiden Elemente Te und Sb, und wenigstens eines der Elemente aus der Gruppe umfassend Nb, Pb, Ta, W, Ti, Al, Zr, Cr, Mn, Ga, Fe, Ru, Co, Rh, Ni, Pd, Pt, La, Bi, B, Ce, Sn, Zn, Si, Na, Li, K, Mg, Ag, Au und In in Kombination enthalten.

Bevorzugt enthält die Kombination dabei aus der letzten Elementgruppe die Elemente Nb, Ta, W und/oder Ti und besonders bevorzugt das Element Nb.

Bevorzugt enthalten die relevanten Multielementoxidaktivmassen die vorgenannte Elementkombination in der Stöchiometrie III

$$Mo_1V_bM^1_cM^2_d \qquad \text{(III)},$$

mit

$M^1$ = Te und/oder Sb,

$M^2$ = wenigstens eines der Elemente aus der Gruppe umfassen Nb, Ta, W, Ti, Al, Zr, Cr, Mn, Ga, Fe, Ru, Co, Rh, Ni, Pd, Pt, La, Bi, Ce, Sn, Zn, Si, Na, Li, K, Mg, Ag, Au und In,

b = 0,01 bis 1,

c = > 0 bis 1, und

d = > 0 bis 1.

Bevorzugt ist $M^1$ = Te und $M^2$ = Nb, Ta, W und/oder Ti. Vorzugsweise ist $M^2$ = Nb.

Der stöchiometrische Koeffizient b beträgt mit Vorteil 0,1 bis 0.6. In entsprechender Weise beläuft sich der Vorzugsbereich für den stöchiometrischen Koeffizienten c auf 0,01 bis 1 bzw. auf 0,05 bis 0,4 und günstige Werte für d betragen 0,01 bis 1 bzw. 0,1 bis 0,6.

Besonders günstig ist es, wenn die stöchiometrischen Koeffizienten b, c und d simultan in den vorgenannten Vorzugsbereichen liegen.

Das Vorgenannte gilt insbesondere dann, wenn die Aktivmasse hinsichtlich ihrer von Sauerstoff verschiedenen Elemente aus einer vorgenannten Elementkombination besteht.

Dies sind dann insbesondere die Multielementoxidaktivmassen der allgemeinen Stöchiometrie IV

$$MoV_bM^1_cM^2_dO_n \qquad \text{(IV)},$$

wobei die Variablen die bezüglich der Stöchiometrie III angeführte Bedeutung aufweisen und n = eine Zahl ist, die durch die Wertigkeit und Häufigkeit der von Sauerstoff verschiedenen Elemente in (IV) bestimmt wird.

Ferner eignet sich das erfindungsgemäße Verfahren bevorzugt zur Herstellung solcher Multielementoxidaktivmassen, die einerseits entweder eine der vorgenannten Elementkombinationen enthalten oder, bezüglich der von Sauerstoff verschiedenen Elemente, aus ihr bestehen und gleichzeitig ein Röntgendiffraktogramm aufweisen, das Beugungsreflexe h und i zeigt, deren Scheitelpunkte bei den Beugungswinkeln (20) 22,2 ± 0,5° (h) und 27,3 ± 0,5° (i) liegen (alle in dieser Schrift auf ein Röntgendiffraktogramm bezogenen Angaben beziehen sich auf ein unter Anwendung von Cu-K$\alpha$-Strahlung als Röntgenstrahlung erzeugtes Röntgendiffraktogramm (Siemens-Diffraktometer Theta-Theta D-5000, Röhrenspannung: 40 kV, Röhrenstrom : 40mA, Aperturblende V20 (variabel), Streustrahlblende V20 (variabel), Sekundärmonochromatorblende (0,1 mm), Detektorblende (0,6 mm), Meßintervall (20): 0,02°, Meßzeit je Schritt: 2,4s, Detektor: Scintillationszählrohr)).

Die Halbwertsbreite dieser Beugungsreflexe kann dabei sehr klein oder auch sehr ausgeprägt sein.

Besonders eignet sich das erfindungsgemäße Verfahren zur Herstellung derjenigen der vorgenannten Multielementoxidaktivmassen, deren Röntgendiffraktogramm zusätzlich zu den Beugungsreflexen h und i einen Beugungsreflexes k aufweist, dessen Scheitelpunkt bei 28,2 ± 0,5° (k) liegt.

Unter den letzteren sind für eine erfindungsgemäße Herstellung wiederum jene bevorzugt, bei denen der Beugungsreflex h innerhalb des Röntgendiffraktogramms der intensitätsstärkste ist, sowie eine Halbwertsbreite von höchstens 0,5° aufweist, und ganz besonders bevorzugt eignet sich das erfindungsgemäße Verfahren für jene, bei denen die Halbwertsbreite des Beugungsreflexes i und des Beugungsreflexes k gleichzeitig jeweils $\leq 1°$ beträgt und die Intensität $P_k$ des Beugungsreflexes k und die Intensität $P_i$ des Beugungsreflexes i die Beziehung $0,2 \leq R \leq 0,85$, besser $0,3 \leq R \leq 0,85$, bevorzugt $0,4 \leq R \leq 0,85$, besonders bevorzugt $0,65 \leq R \leq 0,85$, noch mehr bevorzugt $0,67 \leq R \leq 0,75$ und ganz besonders bevorzugt R = 0,70 bis 0,75 bzw. R = 0,72 erfüllen, in der R das durch die Formel

$$R = P_i/(P_i + P_k)$$

definierte Intensitätsverhältnis ist. Bevorzugt weisen die vorgenannten Röntgendiffraktogramme keinen Beugungsreflex auf, dessen Maximum bei $2\Theta = 50 \pm 0,3°$ liegt.

Die Definition der Intensität eines Beugungsreflexes im Röntgendiffraktogramm bezieht sich in dieser Schrift auf die in der DE-A 19835247, der DE-A 10122027, sowie die in der DE-A 10051419 und DE-A 10046672 niedergelegte Definition. Das gleiche gilt für die Definition der Halbwertsbreite.

Neben den Beugungsreflexen h, i und k enthalten die vorgenannten Röntgendiffraktogramme von erfindungsgemäß vorteilhaft herzustellenden Multielementoxidaktivmassen noch weitere Beugungsreflexe, deren Scheitepunkte bei den nachfolgenden Beugungswinkeln (20) liegen:

9,0 $\pm$ 0,4° (l)
6,7 $\pm$ 0,4° (o) und
7,9 $\pm$ 0,4° (p).

Günstig ist es ferner, wenn das Röntgendiffraktogramm zusätzlich einen Beugungsreflex enthält, dessen Scheitelpunkt beim Beugungswinkel $(2\Theta) = 45,2 \pm 0,4°$ (q) liegt.

Häufig enthält das Röntgendiffraktogramm auch noch die Reflexe 29,2 $\pm$ 0,4° (m) und 35,4 $\pm$ 0,4° (n).

Es ist ferner günstig, wenn die in den Formeln III und IV definierten Elementkombinationen als reine i-Phase vorliegen. Enthält die katalytisch aktive Oxidmasse auch noch k-Phase, enthält ihr Röntgendiffraktogramm neben den oben genannten noch weitere Beugungsreflexe, deren Scheitelpunkte bei den nachfolgenden Beugungswinkeln $(2\Theta)$ liegen: 36,2 $\pm$ 0,4° (m) und 50 $\pm$ 0,4° (die Begriffe i- und k-Phase werden in dieser Schrift wie in der DE-A 10122027 und DE-A 10119933 festgelegt verwendet).

Ordnet man dem Beugungsreflex h die Intensität 100 zu, ist es erfindungsgemäß günstig, wenn die Beugungsreflexe i, l, m, n, o, p, q in der gleichen Intensitätsskala die nachfolgenden Intensitäten aufweisen:

i:    5 bis 95, häufig 5 bis 80, teilweise 10 bis 60;
l:    1 bis 30;
m:    1 bis 40;
n:    1 bis 40;
o:    1 bis 30;
p:    1 bis 30 und
q:    5 bis 60.

Enthält das Röntgendiffraktogramm von den vorgenannten zusätzlichen Beugungsreflexen, ist die Halbwertsbreite derselben in der Regel $\leq 1°$.

Die spezifische Oberfläche von erfindungsgemäß herzustellenden Multielementoxidaktivmassen der allgemeinen Formel IV oder von Multielementoxidaktivmassen, die Elementkombinationen der allgemeinen Formel III enthalten beträgt vielfach 1 bis 30 m$^2$/g (BET-Oberfläche, Stickstoff), vor allem dann, wenn ihr Röntgendiffraktogramm wie beschrieben gestaltet ist.

Die Herstellung der Vorläufermassen der wie beschrieben erhältlichen Multielementoxidaktivmassen findet sich im im Zusammenhang mit diesen zitierten Stand der Technik. Dazu zählen insbesondere die DE-A 10122027, die DE-A 10119933, DE-A 10033121, die EP-A 1192987, die DE-A 10029338, die JP-A 2000-143244, die EP-A 962253, die EP-A 895809, die DE-A 19835247, die WO 00/29105, die WO 00/29106, die EP-A 529853 und die EP-A 608838 (in allen Ausführungsbeispielen der beiden letztgenannten Schriften ist als Trocknungsmethode die Sprühtrocknung anzuwenden; z.B. mit einer Eingangstemperatur von 300 bis 350°C und einer Ausgangstemperatur von 100 bis 150°C; Gegenstrom oder Gleichstrom). Die Bedingungen der thermischen Behandlung dieser Vorläufermassen findet sich ebenfalls in diesen Schriften.

Die beschriebenen Multielementoxidativmassen können als solche (d.h. in Pulverform) oder zu geeigneten Geometrien geformt (vgl. z.B. die Schalenkatalysatoren der DE-A 10051419 sowie die geometrischen Varianten der DE-A 10122027) für die gasphasenkatalytische Partialoxidation und/oder Ammoxidation von niederen Kohlenwasserstoffen und niederen organischen Verbindungen wie Propylen, Propan und Acrolein eingesetzt werden. Sie eignen sich insbesondere zur Herstellung von Acrolein und/oder Acrylsäure sowie zur Herstellung von Acrylnitril aus Propan und/oder Propen. Die Reaktionsbedingungen enthält ebenfalls der zitierte Stand der Technik.

Das erfindungsgemäße Verfahren gestattet nun auch eine regelmäßige Beschickung von Rohrbündelreaktoren (mit Katalysatoren, die als Aktivmasse die besagten katalytisch aktiven Multielementoxidmassen enthalten, die die Elemente Mo, V, wenigstens eines der beiden Elemente Te und Sb und wenigstens eines der Elemente aus der Gruppe umfassend Nb, Ta etc. in Kombination enthalten) mit 5000 bis 40000 Kontaktrohren, die so beschaffen sind, dass bei einer willkürlich herausgegriffenen Stichprobe von 12 Kontaktrohren der Unterschied zwischen der arithmetisch mittleren Aktivität und der höchsten bzw. kleinsten Aktivität nicht mehr als 8°C, häufig nicht mehr als 6°C, vielfach nicht mehr als 4°C und in günstigen Fällen nicht mehr als 2°C beträgt.

Bemerkenswert am erfindungsgemäßen Verfahren ist, dass vorstehendes Ergebnis auch dann erreicht wird, wenn die im Rohrbündelreaktor insgesamt enthaltene Aktivmasse in weniger als 100, bzw. weniger als 75, bzw. weniger als 50 Ansätzen der thermischen Behandlung einer Vorläufermasse hergestellt wurde. Häufig beträgt die Anzahl dieser Ansätze 5 bis 40.

Als Maß für die Aktivität der Kontaktrohrbeschickung wird dabei wiederum die Temperatur verwendet, die ein das einzelne Kontaktrohr umspülende Salzbad (Gemisch aus 53 Gew.-% Kaliumnitrat, 40 Gew.-% Natriumnitrit und 7 Gew.-% Natriumnitrat) aufweisen muß, damit bei einmaligem Durchgang eines Reaktionsgasgemisches aus 4,8 Vol.-% Acrolein, 7 Vol.% Sauerstoff, 10 Vol.% Wasserdampf und 78,2 Vol.% Stickstoff (bei einer Belastung der Katalysatorbeschickung mit 85 Nl Acrotein/l Katalysatorbeschickung x h) durch das beschickte Katalysatorrohr ein Acroleinumsatz von 97 mol-% erzielt wird (unter "l Katalysatorbeschickung" werden dabei die Volumina innerhalb des Katalysatorrohres nicht mit einbezogen, wo sich reine Vor- und Nachschüttungen aus Inertmaterial befinden, sondern nur die Schüttvolumina, die Katalysatorformkörper (gegebenenfalls mit Inertmaterial verdünnt) enthalten).

Eine solche Beschickung von Kontaktrohren ist vor allem dann wichtig, wenn der Rohrbündelreaktor bei einer Acrolein-Belastung der Katalysatorbeschickung betrieben wird, die $\geq$ 135 Nl/l•h, oder $\geq$ 150 Nl/l•h, oder $\geq$ 170 Nl/l•h, oder $\geq$ 200 Nl/l•h, oder $\geq$ 240 Nl/l•h beträgt. In der Regel wird sie $\leq$350 Nl/l•h, bzw. $\leq$300 Nl/l•h oder $\leq$250 Nl/l•h betragen.

Ausführungsbeispiel 1

A) Herstellung einer Vorläufermasse zum Zweck der Erzeugung einer Multielementoxidmasse der Stöchiometrie $Mo_{12}V_3W_{1,2}Cu_{2,4}O_x$

16,3 kg Kupfer(II)acetathydrat (Gehalt: 40,0 Gew.-% CuO) wurden in 274 l Wasser der Temperatur 25°C unter Rühren gelöst. Es wurde eine klare Lösung 1 erhalten.

Räumlich getrennt davon wurden 614 l Wasser auf 40°C erwärmt und 73 kg Ammoniumheptamolybdattetrahydrat (81,5 Gew.-% $MoO_3$) unter Beibehalt der 40°C eingerührt. Dann wurde unter Rühren innerhalb von 30 min auf 90°C erwärmt und unter Beibehalt dieser Temperatur nacheinander und in der genannten Reihenfolge 11,3 kg Ammoniummetavanadat sowie 10,7 kg Ammoniumparawolframattheptahydrat (88,9 Gew.-% $WO_3$) eingerührt. Es wurde eine klare Lösung 2 erhalten.

Die Lösung 2 wurde auf 80°C abgekühlt und anschließend die Lösung 1 in die Lösung 2 eingerührt. Das erhaltene Gemisch wurde mit 130 l einer 25gew.%-igen wässrigen $NH_3$-Lösung versetzt, die eine Temperatur von 25°C aufwies. Unter Rühren entstand eine klare Lösung, die kurzzeitig eine Temperatur von 65°C und einen pH-Wert von 8,5 aufwies. Dieser wurden nochmals 20 l Wasser der Temperatur 25°C zugegeben. Danach stieg die Temperatur der resultierenden Lösung wieder auf 80°C an und diese wurde dann mit einem Sprühtrockner der Fa. Niro-Atomizer (Kopenhagen) vom Typ S-50-N/R sprühgetrocknet (Gaseintrittstemperatur: 350°C, Gasaustrittstemperatur: 110°C). Das Sprühpulver wies einen Partikeldurchmesser von 2 bis 50 $\mu$m auf. 60 kg von so erhaltenem Sprühpulver wurden in einen Kneter der Fa. AMK (Aachener Misch- und Knetmaschinen Fabrik) vom Typ VM 160 (Sigma Schaufeln) dosiert und unter Zusatz von 5,5 l Essigsäure ($\approx$100gew.-%ig, Eisessig) und 5,2 l Wasser geknetet (Drehzahl der Schnecke: 20 Upm). Nach einer Knetdauer von 4 bis 5 Minuten wurden weitere 6,5 l Wasser zugegeben und der Knetprozess bis zum Ablauf von 30 Minuten fortgesetzt (Knettemperatur ca. 40 bis 50°C). Danach wurde das Knetgut in einen Extruder entleert und mittels des Extruders (Fa. Bonnot Company (Ohio), Typ: G 103-10/D7A-572K (6" Extruder W Packer)) zu Strängen (Länge: 1-10 cm; Durchmesser 6 mm) geformt. Auf dem Bandtrockner wurden die Stränge 1 h bei einer Temperatur von 120°C (Materialguttemperatur) getrocknet. Die getrockneten Stränglinge bildeten die nach dem erfindungsgemäßen Verfahren thermisch zu behandelnde Vorläufermasse.

B) Herstellung der katalytischen Aktivmasse durch erfindungsgemäße thermische Behandlung in einer Drehrohrofenvorrichtung

Die Durchführung der thermischen Behandlung erfolgte in einer Drehrohrofenvorrichtung gemäß Figur 1 mit den Ausmaßen und Hilfselementen gemäß der beispielhaften Ausführungsform in der Beschreibung dieser Schrift und unter den folgenden Bedingungen:

- die thermische Behandlung erfolgte diskontinuierlich mit einer Materialgutmenge von 300 kg, die wie wie in A) beschrieben hergestellt worden war;

- der Neigungswinkel des Drehrohres zur Horizontalen betrug $\approx$ 0°;

- das Drehrohr rotierte mit 1,5 Umdrehungen/min rechts herum;

- während der gesamten thermischen Behandlung wurde durch das Drehrohr ein Gasstrom von 205 Nm$^3$/h geführt, der (nach Verdrängung der ursprünglich enthaltenen Luft) wie folgt zusammengesetzt war und an seinem Ausgang aus dem Drehrohr durch weitere 25 Nm$^3$/h Sperrgasstickstoff ergänzt wurde:

80 Nm$^3$/h  zusammengesetzt aus Grundlast-Stickstoff (20) und im Drehrohr freigesetzten Gasen,
25 Nm$^3$/h  Sperrgasstickstoff (11),
30 Nm$^3$/h  Luft (Splitter (21)); und
70 Nm$^3$/h  rezirculiertes Kreisgas (19).

Der Sperrgas-Stickstoff wurde mit einer Temperatur von 25°C zugeführt. Das Gemisch der anderen Gasströme wurde aus dem Erhitzer kommend jeweils mit der Temperatur ins Drehrohr geführt, die das Materialgut im Drehrohr jeweils aufwies.

- innerhalb von 10 h wurde die Materialguttemperatur von 25°C im wesentlichen linear auf 300°C erhitzt; anschließend wurde die Materialguttemperatur innerhalb von 2 h im wesentlichen linear auf 360°C erhitzt; nachfolgend wurde die Materialguttemperatur innerhalb von 7 h im wesentlichen linear auf 350°C gesenkt; dann wurde die Materialguttemperatur innerhalb von 2 h im wesentlichen linear auf 420°C erhöht und diese Materialguttemperatur während 30 min gehalten;

- dann wurden im durch das Drehrohr geführten Gasstrom die 30 Nm$^3$/h Luft durch eine entsprechende Erhöhung des Grundlast-Stickstoff ersetzt (wodurch der Vorgang der eigentlichen thermischen Behandlung beendet wurde), die Beheizung des Drehrohres abgeschaltet und das Materialgut durch Einschalten der Schnellkühlung des Drehrohres durch Ansaugen von Umgebungsluft innerhalb von 2 h auf eine unterhalb von 100°C liegende Temperatur und schließlich auf Umgebungstemperatur abgekühlt; der Gasstrom wurde dem Drehrohr dabei mit einer Temperatur von 25°C zugeführt;

- während der gesamten thermischen Behandlung lag der Druck (unmittelbar) hinter dem Drehrohrausgangs des Gasstroms 0,2 mbar unterhalb des Außendrucks.

Der Sauerstoffgehalt der Gasatmosphäre im Drehrohrofen betrug in allen Phasen der thermischen Behandlung 2,99 Vol.%. Über die Gesamtdauer der reduktiven thermischen Behandlung arithmetisch gemittelt lag die Ammoniakkonzentration der Gasatmosphäre im Drehrohrofen bei 4 Vol.-%.

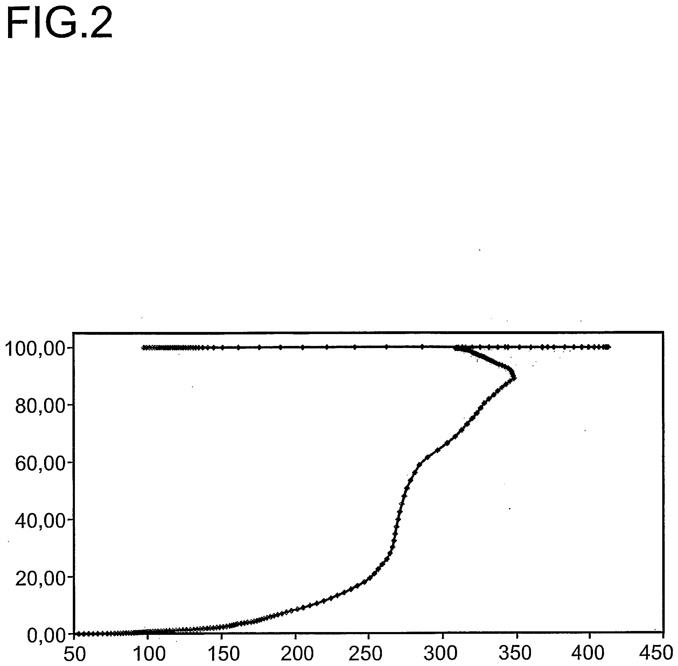

Fig. 2  zeigt die als Funktion der Materialguttemperatur in °C aus der Vorläufermassen freigesetzte Menge an Ammoniak als prozentualen Anteil der im Gesamtverlauf der thermischen Behandlung aus der Vorläufermasse insgesamt freigesetzten Ammoniakmenge.

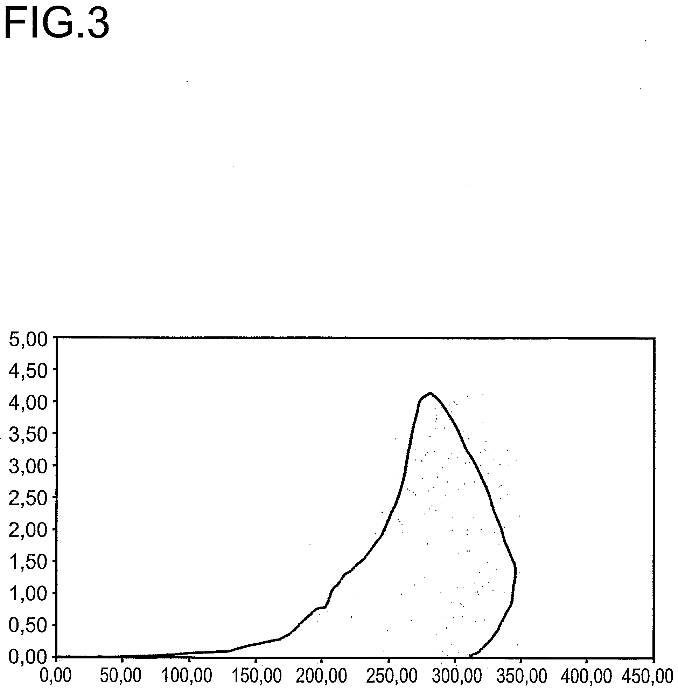

Fig. 3  zeigt die Ammoniakkonzentration der Atmosphäre in Vol.-% in der die thermische Behandlung erfolgte in Abhängigkeit von der Materialguttemperatur in °C während der thermischen Behandlung.

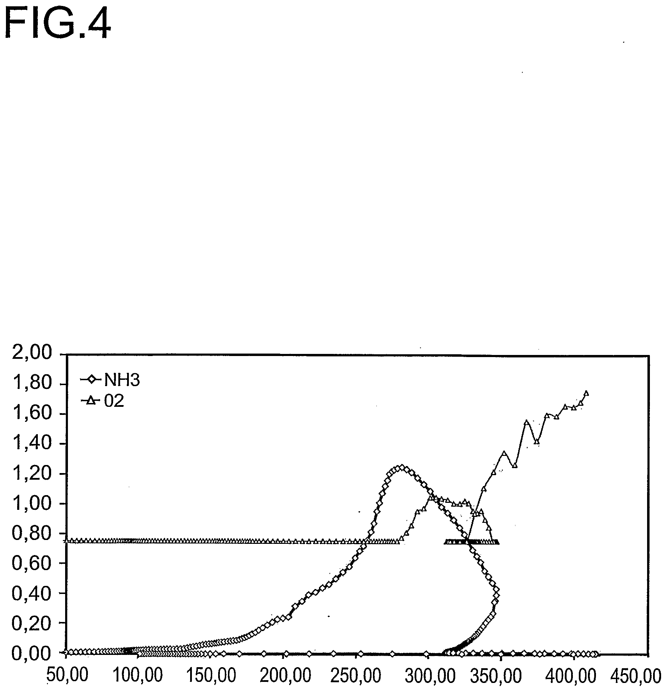

Fig. 4  zeigt in Abhängigkeit von der Materialguttemperatur die molaren Mengen an molekularem Sauerstoff sowie an Ammoniak, die je kg Vorläufermasse und Stunde über die thermische Behandlung mit dem Gasstrom ins Drehrohr geführt wurden.

C) Formgebung der Multimetalloxidaktivmasse

Das unter "B)" erhaltene katalytisch aktive Material wurde mittels einer Biplexquerstromsichtmühle (BQ 500) (Fa. Hosokawa-Alpine Augsburg) zu einem feinteiligen Pulver gemahlen, von dem 50 % der Pulverpartikel ein Sieb der Maschenweite 1 bis 10 $\mu$m passierten und dessen Anteil an Partikeln mit einer Längstausdehnung oberhalb von 50 $\mu$m weniger als 1 % betrug.

Mittels des gemahlenen Pulvers wurden wie in S1 der EP-B 714700 ringförmige Trägerkörper (7 mm Außendurchmesser, 3 mm Länge, 4 mm Innendurchmesser, Steatit C220 der Fa. CeramTec mit einer Oberflächenrauhigkeit R$_z$ von 45 $\mu$m) beschichtet. Bindemittel war eine wässrige Lösung aus 75 Gew.-% Wasser und 25 Gew.-% Glycerin.

Der Aktivmassenanteil der resultierenden Schalenkatalysatoren wurde jedoch im Unterschied zu vorgenanntem Beispiel S1 zu 20 Gew.-% (bezogen auf das Gesamtgewicht aus Trägerkörper und Aktivmasse) gewählt. Das Mengenverhältnis von Pulver und Bindemittel wurde proportional angepasst.

D) Testung der Schalenkatalysatoren

Die Schalenkatalysatoren wurden wie folgt in einem von einem Salzbad (Gemisch aus 53 Gew.-% Kaliumnitrat, 40 Gew.-% Natriumnitrit und 7 Gew.-% Natriumnitrat) umspülten Modellkontaktrohr getestet:

Modellkontaktrohr: V2A-Stahl, 2 mm Wandstärke, 26 mm Innendurchmesser, zentriert eine Thermohülse (zur Aufnahme eines Thermoelements) des Außendurchmessers 4 mm, 1,56 l des freien Modellkontaktrohrraumes wurden mit dem Schalenkatalysator gefüllt.

Das Reaktionsgasgemisch wies folgende Ausgangszusammensetzung auf:

4,8 Vol.-% Acrolein, 7 Vol.-% Sauerstoff, 10 Vol.-% Wasserdampf, 78,2 Vol.-% Stickstoff.

Das Modellkontaktrohr wurde mit 2800 Nl/h an Reaktionsgasausgangsgemisch belastet. Dem entspricht eine Belastung der Katalysatorbeschickung von 86 Nl/l•h. Die Temperatur des Salzbades wurde so eingestellt, dass bei einfachem Durchgang ein Acroleinumsatz von 97 mol-% resultierte.
In zehn voneinander unabhängigen Versuchen wurden jeweils 12 wie beschrieben beschickte Modellkontaktrohre miteinander verglichen.
In allen Fällen lag die für den geforderten Acroleinumsatz benötigte Salzbadtemperatur im Intervall 257 ± 4°C. Die Selektivität der Acrylsäurebildung lag in allen Fällen bei etwa 94,8 mol-%.

Ausführungsbeispiel 2

Alles wurde wie im Ausführungsbeispiel 1 durchgeführt. Die Formgebung der Multimetalloxidaktivmasse erfolgte allerdings wie folgt:

70 kg ringförmige Trägerkörper (7,1 mm Außendurchmesser, 3,2 mm Länge, 4,0 mm Innendurchmesser; Steatit des Typs C220 der Fa. CeramTec mit einer Oberflächenrauhigkeit $R_z$ von 45 $\mu$m und einem auf das Volumen des Trägerkörpers bezogenen Porengesamtvolumen ≤ 1 Vol.-%) wurden in einen Dragierkessel (Neigungswinkel 90°; Hicoater der Fa. Lödige, DE) von 200 l Innenvolumen gefüllt. Anschließend wurde der Dragierkessel mit 16 U/min in Rotation versetzt. Über eine Düse wurden innerhalb von 25 min 3,8 bis 4,2 Liter einer wässrigen Lösung aus 75 Gew.-% Wasser und 25 Gew.-% Glycerin auf die Trägerkörper aufgesprüht. Gleichzeitig wurden im selben Zeitraum 18,1 kg der gemahlenen Multimetalloxidaktivmasse (deren spezifische Oberfläche betrug 13,8 m$^2$/g) über eine Schüttelrinne außerhalb des Sprühkegels der Zerstäuberdüse kontinuierlich zudosiert. Während der Beschichtung wurde das zugeführte Pulver vollständig auf die Oberfläche des Trägerkörpers aufgenommen, eine Agglomeration der feinteiligen oxidischen Aktivmasse wurde nicht beobachtet. Nach beendeter Zugabe von Aktivmassenpulver und Wasser wurde bei einer Drehgeschwindigkeit von 2 U/min 40 min (alternativ 15 bis 60 min) 100°C (alternativ 80 bis 120°C) heiße Luft (ca. 400 m$^3$/h) in den Dragierkessel geblasen. Es wurden ringförmige Schalenkatalysatoren erhalten, deren Anteil an oxidischer Aktivmasse, bezogen auf die Gesamtmasse, 20 Gew.-% betrug. Die Schalendicke lag, sowohl über die Oberfläche eines Trägerkörpers als auch über die Oberfläche verschiedener Trägerkörper betrachtet, bei 170 ± 50 $\mu$m.

Die Testung der Schalenkatalysatoren erfolgte wie im Ausführungsbeispiel 1. Die resultierenden Ergebnisse entsprachen den in Ausführungsbeispiel 1 erzielten Resultaten.

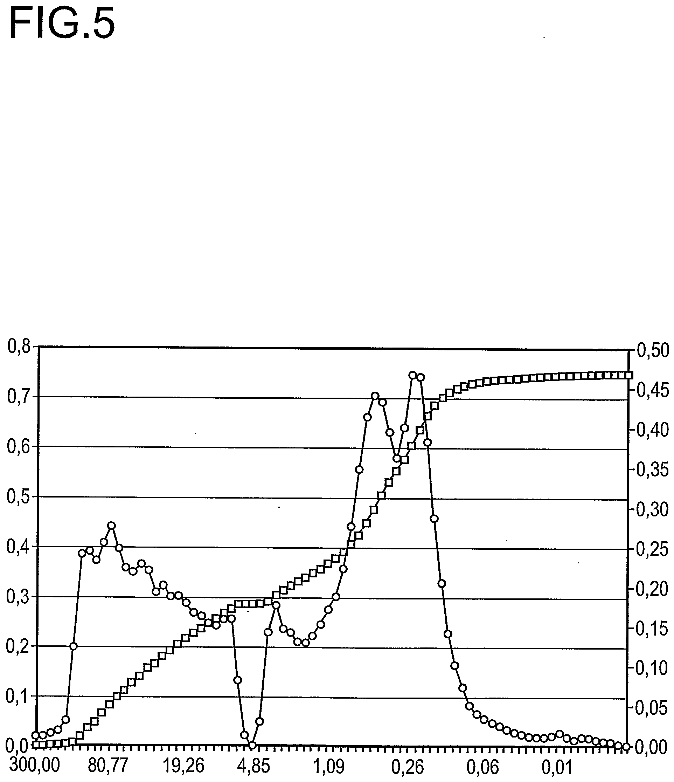

Figur 5 zeigt außerdem die Porenverteilung des gemahlenen Aktivmassenpulvers vor seiner Formgebung. Auf der Abszisse ist der Porendurchmesser in $\mu$m aufgetragen (logarithmische Skala).
Auf der rechten Ordinate ist der Logarithmus des differentiellen Beitrags in ml/g des jeweilige Porendurchmessers zum Porengesamtvolumen aufgetragen (Kurve O). Das Maximum weist den Porendurchmesser mit dem größten Beitrag zum Porengesamtvolumen aus. Auf der linken Ordinate ist in ml/g das Integral über die individuellen Beiträge der einzelnen Porendurchmesser zum Porengesamtvolumen aufgetragen (Kurve □). Der Endpunkt ist das Porengesamtvolumen (alle Angaben in dieser Schrift zu Bestimmungen von Porengesamtvolumina sowie von Durchmesserverteilungen auf diese Porengesamtvolumina beziehen sich, soweit nichts anderes erwähnt wird, auf Bestimmungen mit der Methode der Quecksilberporosimetrie in Anwendung des Gerätes Auto Pore 9220 der Fa. Micromeritics GmbH, 4040 Neuß, DE (Bandbreite 30 Å bis 0,3 mm); alle Angaben in dieser Schrift zu Bestimmungen von spezifischen Oberflächen bzw. von Mikroporenvolumina beziehen sich auf Bestimmungen nach DIN 66131 (Bestimmung der spezifischen Oberfläche von Feststoffen durch Gasadsorption (N$_2$) nach Brunauer-Emmet-Teller (BET)).

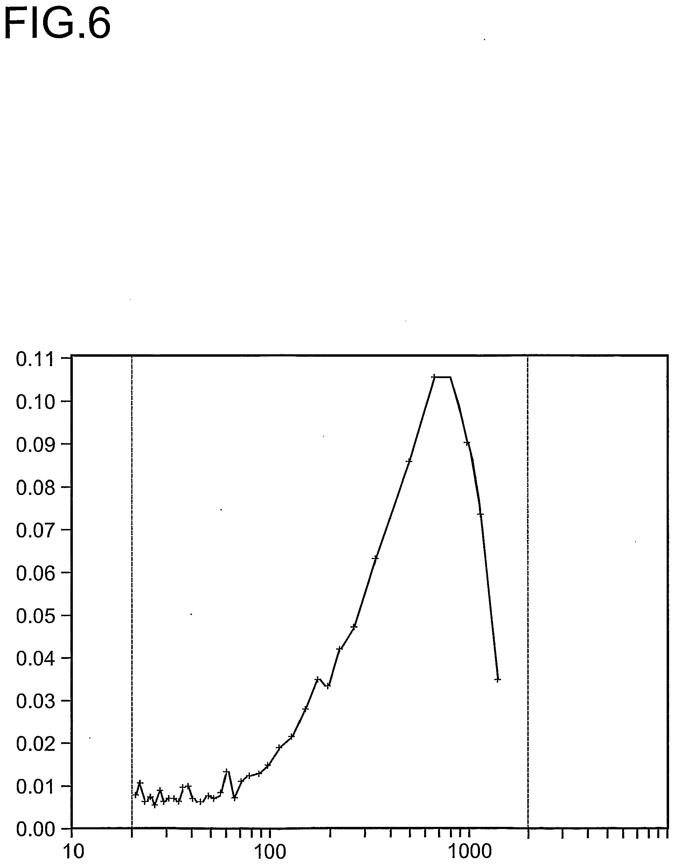

Figur 6 zeigt für das Aktivmassenpulver vor seiner Formgebung in ml/g (Ordinate) die individuellen Beiträge der einzelnen Porendurchmesser (Abszisse, in Angström, logarithmische Skala) im Mikroporenbereich zum Porengesamtvolumen.

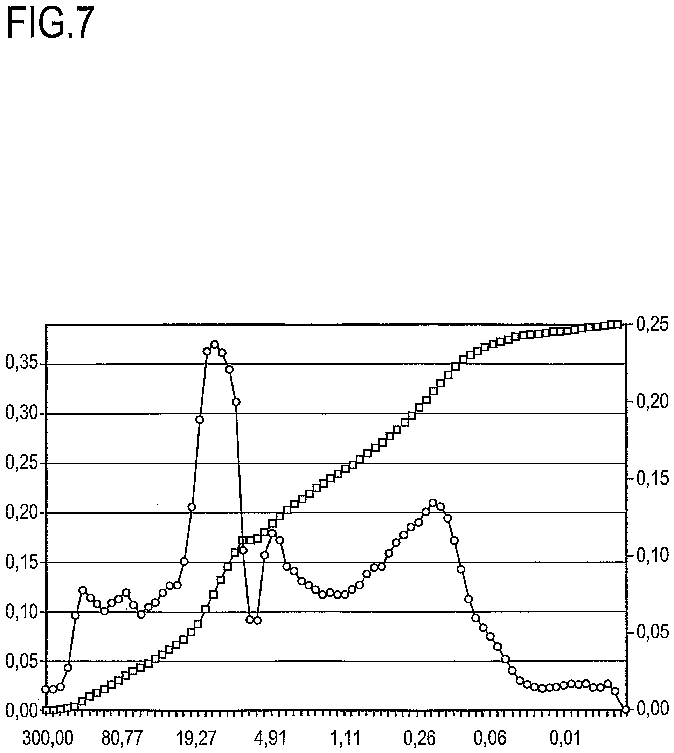

Figur 7 zeigt das gleiche wie Figur 5, jedoch für vom ringförmigen Schalenkatalysator durch mechanisches Abkratzen nachträglich gelöste Multimetalloxidaktivmasse (ihre spezifische Oberfläche betrug 12,9 $m^2$/g).

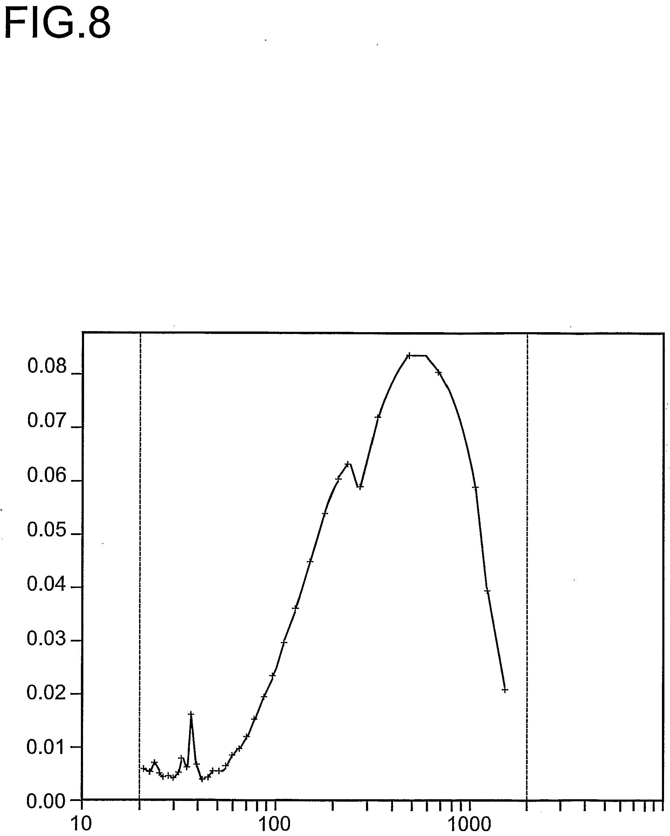

Figur 8 zeigt das gleiche wie Figur 6, jedoch für vom ringförmigen Schalenkatalysator durch mechanisches Abkratzen nachträglich gelöste Multimetalloxidaktivmasse.

Ausführungsbeispiel 3

Alles wurde wie im Ausführungsbeispiel 1 durchgeführt. Die Formgebung der Multimetalloxidaktivmasse erfolgte allerdings wie folgt:

70 kg kugelförmige Trägerkörper (Durchmesser 4 bis 5 mm; Steatit des Typs C220 der Fa. CeramTec mit einer Oberflächenrauhigkeit $R_z$ von 45 $\mu$m und einem auf das Volumen des Trägerkörpers bezogenen Porengesamtvolumen $\leq$ 1 Vol.-%) wurde in einen Dragierkessel (Neigungswinkel 90°; Hicoater der Fa. Lödige, DE) von 200 l Innenvolumen gefüllt. Anschließend wurde der Dragierkessel mit 16 U/min in Rotation versetzt. Über eine Düse wurden innerhalb von 25 min 2, 8 bis 3,3 Liter Wasser auf die Trägerkörper aufgesprüht. Gleichzeitig wurden im selben Zeitraum 14,8 kg der gemahlenen Multimetalloxidaktivmasse über eine Schüttelrinne außerhalb des Sprühkegels der Zerstäuberdüse kontinuierlich zudosiert. Während der Beschichtung wurde das zugeführte Pulver vollständig auf die Oberfläche der Trägerkörper aufgenommen, eine Agglomeration der feinteiligen oxidischen Aktivmasse wurde nicht beobachtet. Nach beendeter Zugabe von Pulver und Wasser wurde bei einer Drehgeschwindigkeit von 2 U/min 40 min (alternativ 15 bis 60 min) 100°C (alternativ 80 bis 120°C) heiße Luft (ca. 400 $m^3$/h) in den Dragierkessel geblasen. Es wurden kugelförmige Schalenkatalysatoren erhalten, deren Anteil an oxidischer Aktivmasse, bezogen auf die Gesamtmasse, 17 Gew.-% betrug. Die Schalendicke lag, sowohl über die Oberfläche eines Trägerkörpers als auch über die Oberfläche verschiedener Trägerkörper betrachtet, bei 160 $\pm$ 50 $\mu$m.

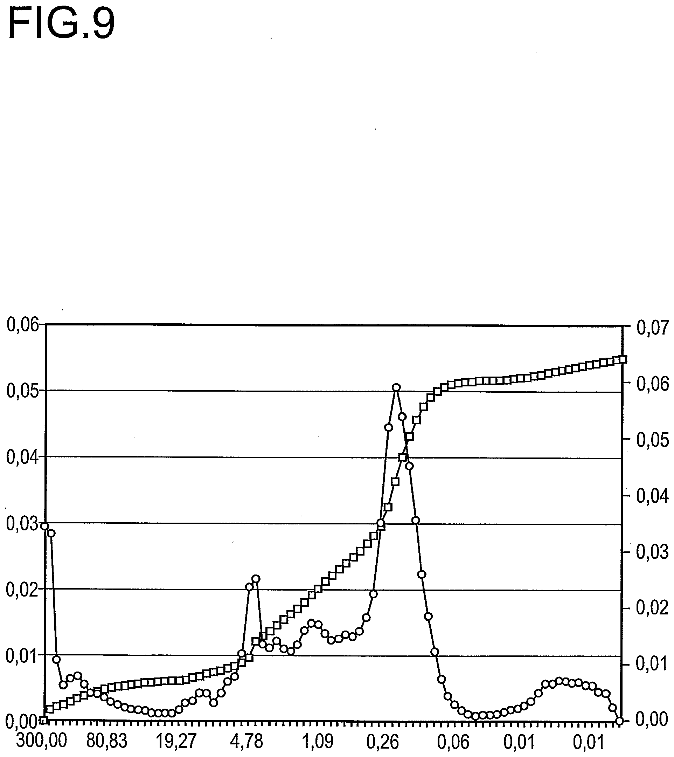

Figur 9 zeigt das Analogon zu Figur 7 (die spezifische Oberfläche der abgekratzten Multimetalloxidaktivmasse betrug 15 $m^2$/g).

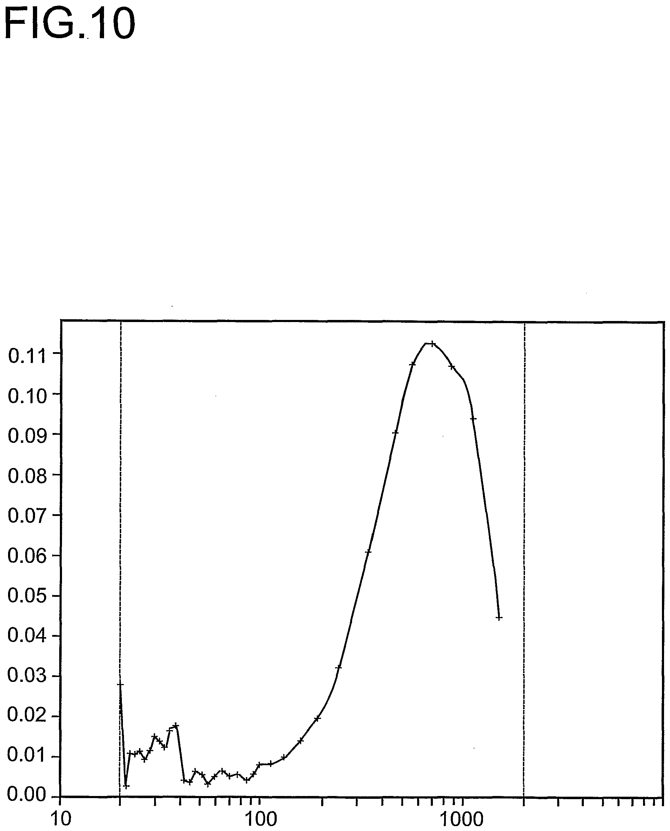

Figur 10 zeigt das Analogon zur Figur 8.

Die Testung des kugelförmigen Schalenkatalysators erfolgte wie in Herstellungsbeispiel 1 für den ringförmigen Schalenkatalysator beschrieben.

Alle in dieser Schrift beispielhaft hergestellten Schalenkatalysatoren eignen sich insbesondere für Acroleinpartialoxidationen bei hohen Acroleinbelastungen der Katalysatorbeschickung (z.B. $\geq$ 135 Nl/l•h bis 350 Nl/l•h).

## Patentansprüche

1. Verfahren zur thermischen Behandlung der Vorläufermasse einer katalytischen Aktivmasse, die eine Multielementoxidmasse ist, die wenigstens eines der Elemente Nb und W sowie die Elemente Mo und V enthält, wobei der molare Anteil des Elements Mo an der Gesamtmenge aller von Sauerstoff verschiedenen Elemente der katalytischen Aktivmasse 20 mol-% bis 80 mol-% beträgt, das molare Verhältnis von in der katalytisch aktiven Multielementoxidmasse enthaltenem Mo zu in der katalytisch aktiven Multielementoxidmasse enthaltenem V, Mo/V, 15:1 bis 1:1 und das entsprechende molare Verhältnis Mo/(Gesamtmenge aus W und Nb) 80:1 bis 1:4 beträgt, in einem von einem Gasstrom durchströmten Drehrohrofen bei einer Materialguttemperatur von 300 bis 450°C in einer $O_2$ und $NH_3$ aufweisenden Gasatmosphäre, dadurchgekennzeichnet, dass

man als Vorläufermasse aus Ausgangsverbi ndungen, die die von Sauerstoff verschiedenen elementaren Konstituenten der Multielementoxidmasse als Bestandteile enthalten, ein inniges, auch Ammoniumionen enthaltendes, Trockengemisch herstellt,
die Gasatmosphäre, in der diethermische Behandlung erfolgt, eine solche ist, die
zu jedem Zeitpunkt 0,5 bis 4 Vol-% $O_2$,
über die Gesamtdauer der reduktiven thermischen Behandlung gemittelt 1 bis 8 Vol.% $NH_3$, sowie Wasserdampf und/oder Inertgas als Restmengen enthält,
der $NH_3$-Gehalt der Atmosphäre während der thermischen Behandlung ein Maximum durchläuft, das unterhalb von 20 Vol.-% liegt, und
wenigstens eine Teilmenge des den Drehrohrofen durchströmenden Gasstroms im Kreis geführt wird und keine externe Zufuhr von $NH_3$ erfolgt.

2. Verfahren nach Anspruch 1, **dadurch gekennzeichnet, dass** die Multielementoxidmasse zusätzlich das Element Cu enthält.

**3.** Verfahren nach Anspruch 1 oder 2, **dadurch gekennzeichnet, dass** die Multielementoxidmasse nur aus Nb und/oder W sowie Mo und V und gegebenenfalls Cu besteht.

**4.** Verwendung von Multielementoxidmassen, die nach einem Verfahren gemäß einem der Ansprüche 1 bis 3 hergestellt worden sind, als Aktivmasse für Katalysatoren zur heterogen katalysierten Gasphasenoxidation von Acrolein zu Acrylsäure.

**5.** Heterogen katalysierte Gasphasenoxidation von Acrolein zu Acrylsäure, **dadurch gekennzeichnet, dass** die Aktivmasse der Katalysatoren eine Multielementoxidmasse ist, die nach einem Verfahren gemäß einem der Ansprüche 1 bis 3 hergestellt worden ist.

**6.** Gasphasenoxidation gemäß Anspruch 5, **dadurch gekennzeichnet, dass** die Gasphasenoxidation in einem Rohrbündelreaktor mit 5000 bis 40000 Kontaktrohren durchgeführt wird.

**Claims**

**1.** A process for the thermal treatment of the precursor composition of a catalytic active composition which is a multielement oxide composition comprising at least one of the elements Nb and W and also the elements Mo and V, where the molar proportion of the element Mo based on the total amount of all elements other than oxygen in the catalytic active composition is from 20 mol% to 80 mol%, the molar ratio of Mo comprised in the catalytically active multielement oxide composition to V comprised in the catalytically active multielement oxide composition, Mo/V, is from 15:1 to 1:1 and the corresponding molar ratio of Mo/(total amount of W and Nb) is from 80:1 to 1:4, at a temperature of the material of from 300 to 450°C in a gas atmosphere comprising $O_2$ and $NH_3$ in a rotary tube furnace through which a gas stream flows, wherein

an intimate dry mixture also comprising ammonium ions is produced as precursor composition from starting compounds comprising the elemental constituents other than oxygen of the multielement oxide composition as constituent,

the gas atmosphere in which the thermal treatment is carried out is an atmosphere which

further comprises from 0.5 to 4% by volume of $O_2$ at every point in time, from 1 to 8% by volume of $NH_3$ averaged over the total duration of the reductive thermal treatment and
water vapor and/or inert gas as balance,

the $NH_3$ content of the atmosphere during the thermal treatment goes through a maximum which is below 20% by volume and
at least a substream of the gas stream flowing through the rotary tube furnace is circulated and no external introduction of $NH_3$ is carried out.

**2.** The process as claimed in claim 1, wherein the multielement oxide composition additionally comprises the element Cu.

**3.** The process as claimed in claim 1 or 2, wherein the multielement oxide composition consists only of Nb and/or W and also Mo and V and optionally Cu.

**4.** The use of multielement oxide compositions which have been produced by a process as claimed in any of claims 1 to 3 as active composition for catalysts for the heterogeneously catalyzed gas-phase oxidation of acrolein to acrylic acid.

**5.** A heterogeneously catalyzed gas-phase oxidation of acrolein to acrylic acid, wherein the active composition of the catalysts is a multielement oxide composition which has been produced by a process as claimed in any of claims 1 to 3.

**6.** The gas-phase oxidation as claimed in claim 5, wherein the gas-phase oxidation is carried out in a shell-and-tube reactor having from 5000 to 40 000 catalyst tubes.

**Revendications**

1. Procédé pour le traitement thermique de la masse de précurseur d'une masse catalytiquement active, qui est une masse d'oxyde d'éléments multiples, qui contient au moins un des éléments Nb et W ainsi que les éléments Mo et V, la proportion molaire de l'élément Mo à la quantité totale de tous les éléments différents de l'oxygène de la masse catalytiquement active valant 20% en mole à 80% en mole, le rapport molaire du Mo contenu dans la masse d'oxyde d'éléments multiples catalytiquement active au V contenu dans la masse d'oxyde d'éléments multiples catalytiquement active, Mo/V, valant 15:1 à 1:1 et le rapport molaire correspondant Mo/(quantité totale de W et de Nb) valant 80:1 à 1:4, dans un four tubulaire rotatif traversé par un flux gazeux à une température du matériau de 300 à 450°C dans une atmosphère gazeuse présentant $O_2$ et $NH_3$, **caractérisé en ce que**

   on prépare, comme masse de précurseur, à partir de composés de départ, qui contiennent les constituants élémentaires différents de l'oxygène de la masse d'oxyde d'éléments multiples comme constituants, un mélange sec intime, contenant également des ions d'ammonium,

   l'atmosphère gazeuse, dans laquelle a lieu le traitement thermique, est une atmosphère qui contient,

   à tout moment, 0,5 à 4% en volume d'$O_2$,
   sur la durée totale du traitement thermique réducteur, en moyenne 1 à 8% en volume de $NH_3$, ainsi que de la vapeur d'eau et/ou un gaz inerte comme quantités résiduelles,

   la teneur en $NH_3$ de l'atmosphère pendant le traitement thermique passe par un maximum qui se situe en dessous de 20% en volume et
   au moins une quantité partielle du flux gazeux s'écoulant au travers du four tubulaire rotatif est guidée en circulation et aucune alimentation externe en $NH_3$ n'a lieu.

2. Procédé selon la revendication 1, **caractérisé en ce que** la masse d'oxyde d'éléments multiples contient en outre l'élément Cu.

3. Procédé selon la revendication 1 ou 2, **caractérisé en ce que** la masse d'oxyde d'éléments multiples n'est constituée que par du Nb et/ou du W ainsi que par du Mo et du V et le cas échéant par du Cu.

4. Utilisation de masses d'oxyde d'éléments multiples, qui ont été préparées selon un procédé selon l'une quelconque des revendications 1 à 3 comme masse active pour des catalyseurs pour l'oxydation en phase gazeuse, catalysée par voie hétérogène, d'acroléine en acide acrylique.

5. Oxydation en phase gazeuse, catalysée par voie hétérogène, d'acroléine en acide acrylique, **caractérisée en ce que** la masse active des catalyseurs est une masse d'oxyde d'éléments multiples qui a été préparée selon un procédé selon l'une quelconque des revendications 1 à 3.

6. Oxydation en phase gazeuse selon la revendication 5, **caractérisée en ce que** l'oxydation en phase gazeuse est réalisée dans un réacteur à faisceau tubulaire présentant 5.000 à 40.000 tubes de contact.

# FIG.1

# FIG.2

# FIG.3

# FIG.4

# FIG.5

# FIG.6

# FIG.7

# FIG.8

# FIG.9

# FIG.10

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10211275 A
- EP 529853 A
- EP 318295 A
- EP 608838 A
- WO 0196270 A
- EP 731077 A
- EP 1260495 A
- EP 1254709 A
- EP 1192987 A
- EP 962253 A
- EP 1262235 A
- EP 1090684 A
- DE 19835247 A
- EP 895809 A
- DE 10261186 A
- EP 774297 A
- WO 0224620 A
- EP 668104 A
- DE 2161450 A
- EP 724481 A
- EP 714700 A
- DE 10046928 A
- DE 19815281 A
- DE 19855913 A
- EP 72448 B
- DE 2909671 A
- EP 293859 A
- DE 19910508 A
- EP 724481 B
- DE 10307983 A
- DE 19948523 A
- EP 700714 A
- EP 700893 A
- DE 19910506 A
- DE 19948241 A
- DE 2830765 C
- DE 2513405 C
- US 3147084 A
- DE 2201528 A
- EP 383224 A
- DE 2903218 A
- EP 468290 B
- DE 10122027 A
- DE 10051419 A
- DE 10046672 A
- DE 10119933 A
- DE 10033121 A
- DE 10029338 A
- JP 2000143244 A
- WO 0029105 A
- WO 0029106 A
- EP 714700 B